# EUROPEAN PATENT SPECIFICATION

(11) **EP 3 215 844 B1**
(45) Date of publication and mention of the grant of the patent: **28.04.2021**
(21) Application number: 15794313.5
(22) Date of filing: 19.08.2015
(51) Int. Cl.: G01N 33/50, G01N 33/574

(54) **METHODS FOR PREDICTING AND MONITORING CANCER PATIENTS' RESPONSE TO TREATMENT BY MEASURING MYELOID DERIVED SUPPRESSOR CELLS (MDSCS)**
VERFAHREN ZUR VORHERSAGE UND ÜBERWACHUNG DER REAKTION VON KREBSPATIENTEN AUF DIE BEHANDLUNG DURCH MESSUNG MYELOIDER SUPPRESSOR-ZELLEN (MDSCS)
MÉTHODES DE PRÉVISION ET DE SURVEILLANCE DE LA RÉPONSE DE PATIENTS CANCÉREUX À UN TRAITEMENT EN MESURANT LES CELLULES MYÉLOÏDES SUPPRESSIVES (MDSC)

(30) Priority: 19.08.2014 US 201462039156 P; 13.11.2014 US 201462079018 P
(43) Date of publication of application: 13.09.2017
(73) Proprietor: Yissum Research Development Company of the Hebrew University of Jerusalem Ltd, 9139002 Jerusalem (IL); Hadasit Medical Research Services and Development Ltd., 9112001 Jerusalem (IL)
(72) Inventor: BANIYASH, Michal, 9075804 Mevasseret Zion (IL); LOTEM, Michal, 7179902 Makabim-Re'ut (IL); SADE-FELDMAN, Moshe, 7172129 Modiin (IL); KANTERMAN, Julia, 7172129 Modiin (IL)
(74) Representative: Boult Wade Tennant LLP
(86) International application number: PCT/IL2015/050837
(87) International publication number: WO 2016/027273

(56) References cited:
- US-A1- 2012 276 004
- A. J. MONTERO ET AL: "Phase 2 study of neoadjuvant treatment with NOV-002 in combination with doxorubicin and cyclophosphamide followed by docetaxel in patients with HER-2 negative clinical stage II-IIIc breast cancer", BREAST CANCER RESEARCH AND TREATMENT., vol. 132, no. 1, 1 February 2012 (2012-02-01), pages 215-223, XP055238960, US ISSN: 0167-6806, DOI: 10.1007/s10549-011-1889-0
- S. KITANO ET AL: "Computational Algorithm-Driven Evaluation of Monocytic Myeloid-Derived Suppressor Cell Frequency for Prediction of Clinical Outcomes", CANCER IMMUNOLOGY RESEARCH, vol. 2, no. 8, 20 May 2014 (2014-05-20), pages 812-821, XP055238733, US ISSN: 2326-6066, DOI: 10.1158/2326-6066.CIR-14-0013
- CHRISTIANE MEYER ET AL: "Frequencies of circulating MDSC correlate with clinical outcome of melanoma patients treated with ipilimumab", CANCER IMMUNOLOGY, IMMUNOTHERAPY, vol. 63, no. 3, 1 March 2014 (2014-03-01), pages 247-257, XP055166338, ISSN: 0340-7004, DOI: 10.1007/s00262-013-1508-5
- AHMAD A. TARHINI ET AL: "Immune Monitoring of the Circulation and the Tumor Microenvironment in Patients with Regionally Advanced Melanoma Receiving Neoadjuvant Ipilimumab", PLOS ONE, vol. 9, no. 2, 3 February 2014 (2014-02-03), page e87705, XP055238901, DOI: 10.1371/journal.pone.0087705
- BIN ZHANG ET AL: "Circulating and Tumor-Infiltrating Myeloid-Derived Suppressor Cells in Patients with Colorectal Carcinoma", PLOS ONE, vol. 8, no. 2, 20 February 2013 (2013-02-20), page e57114, XP055238993, DOI: 10.1371/journal.pone.0057114
- MELISSA G LECHNER ET AL: "Functional characterization of human Cd33+ And Cd11b+ myeloid-derived suppressor cell subsets induced from peripheral blood mononuclear cells co-cultured with a diverse set of human tumor cell lines", JOURNAL OF TRANSLATIONAL MEDICINE, BIOMED CENTRAL, LONDON, GB, vol. 9, no. 1, 9 June 2011 (2011-06-09), page 90, XP021103820, ISSN: 1479-5876, DOI: 10.1186/1479-5876-9-90
- MANJA IDORN ET AL: "Correlation between frequencies of blood monocytic myeloid-derived suppressor cells, regulatory T cells and negative prognostic markers in patients with castration-resistant metastatic prostate cancer", CANCER IMMUNOLOGY AND IMMUNOTHERAPY, vol. 63, no. 11, 2 August 2014 (2014-08-02), pages 1177-1187, XP055238899, BERLIN, DE ISSN: 0340-7004, DOI: 10.1007/s00262-014-1591-2
- J. KANTERMAN ET AL: "Adverse Immunoregulatory Effects of 5FU and CPT11 Chemotherapy on Myeloid-Derived Suppressor Cells and Colorectal Cancer Outcomes", CANCER RESEARCH, vol. 74, no. 21, 1 November 2014 (2014-11-01), pages 6022-6035, XP055238916, US ISSN: 0008-5472, DOI: 10.1158/0008-5472.CAN-14-0657
- Anonymous: "BD Vacutainer,CPT Mononuclear Cell Preparation,Tube REF 362753", BD Biosciences , Retrieved from the Internet: URL:http://www.bdbiosciences.com/us/applic ations/blood-collection/cell-biomarker-pre servation/bd-vacutainerreg-cpttrade-mononu clear-cell-preparation-tube---sodium-hepar in/p/362753 [retrieved on 2018-10-15]

### SEQUENCE LISTING

<110> Yissum Research Development Company of the Hebrew University of Jerusalem Ltd. Hadasit Medical Research Services & Development Limited
<120> Methods for predicting and monitoring cancer patients' response to treatment by measuring myeloid derived suppressor cells (MDSCs)
<130> 2364192
<150> US 62/039,156
   <151> 2014-08-19
<150> US 62/079,018
   <151> 2014-11-13
<160> 8
<170> PatentIn version 3.5
<210> 1
   <211> 23
   <212> DNA
   <213> Mus musculus
<400> 1
   ggaaatcacc atgccctcta caa 23
<210> 2
   <211> 23
   <212> DNA
   <213> Mus musculus
<400> 2
   atgccacacc cacttttatc acc 23
<210> 3
   <211> 22
   <212> DNA
   <213> Mus musculus
<400> 3
   ggagcgcagc ataaccacca tc 22
<210> 4
   <211> 24
   <212> DNA
   <213> Mus musculus
<400> 4
   gccatcagca tcatacactc ctca 24
<210> 5
   <211> 21
   <212> DNA
   <213> Mus musculus
<400> 5
   gttaagcagt acagccccaa a 21
<210> 6
   <211> 23
   <212> DNA
   <213> Mus musculus
<400> 6
   agggcatatc caacaacaaa ctt 23
<210> 7
   <211> 22
   <212> DNA
   <213> Mus musculus
<400> 7
   gccaccacgc tcttctgtct ac 22
<210> 8
   <211> 22
   <212> DNA
   <213> Mus musculus
<400> 8
   gggtctgggc catagaactg at 22

## Description

### TECHNOLOGICAL FIELD

The invention is in the field of cancer therapy and in particular, the invention concerns methods for determining a cancer patient's therapy, as well as predicting and monitoring a cancer patient's response to therapy with an immunotherapeutic agent.

### BACKGROUND ART

References considered to be relevant as background to the presently disclosed subject matter are listed below:
- WO 2012/0276004
- WO 2012/149416
- WO 2013/050998
- Montero et al, 2012. Breast Canc Res Treat, 132, 215-223
- Kitano et al, 2014. Canc Immunol Res, 8(2), 812-821
- Meyer et al, 2014. Canc Immunol Immunother, 63(3), 247-257
- Tarhini et al, 2014. PlosOne, 9(2), e87705
- Zhang et al, 2013. PlosOne, 8(2), e57114
- Lechner et al, 2011. J Transl Med, 9(1), 90
- Idorn et al, 2014. Canc Immunol Immunother, 63(11), 1177-1187
- US2012/276004

Acknowledgement of the above references herein is not to be inferred as meaning that these are in any way relevant to the patentability of the presently disclosed subject matter.

### BACKGROUND

Many tumors are characterized by chronic inflammation-induced immunosuppression mediated by pro-inflammatory cells and mediators (1-4), which subvert the outcome of anti-cancer therapy. MDSCs are the main cell population causing immunosuppression in numerous cancers including CRC (3, 5-8). MDSCs are immature myeloid cells expanded in the course of chronic inflammation.

Such generated conditions manipulate the host's immune system; suppressing the innate and adaptive immune responses, as reflected by the impaired function of T and NK cells and is associated with down regulated expression of the CD247 and SNX9 molecules. Such chronic inflammation-induced immunosuppressive features act as critical barriers to effective anti-tumor responses and therapies.

WO 2012/0276004 discloses methods for determining the presence of cancer, monitoring cancer progression, cancer relapse, or cancer staging in a subject by evaluating specific MDSC cell populations.

WO 2012/149416 discloses a method of diagnosing, treating, or determining efficacy of treatment of a cancer patient, in particular a lymphoma patient, including a step of assessing the level of MDSC of specific phenotypes in the patient.

WO 2013/050998 discloses a method for determining the efficacy of treatment of a subject suffering from a chronic inflammatory condition comprising determining the level of expression of T cell antigen receptor (TCR) ζ chain (CD247). Optionally, this method may further include determining the MDSC population in the subject.

Montero et al, 2012 discloses a method for determining the efficacy of treatment of a subject suffering from HER2 negative clinical stage IIIc breast cancer comprising the measurement of MDSCs levels in fresh whole blood samples of those patients by employing a "Lin-1-FITC cocktail (CD3/CD16/CD19/CD20/CD56)" for incubation, with subsequently lysis, washing and centrifugation step in said samples and in the end calculates absolute numbers of MDSCs.

Kitano et al, 2014 discloses a method of evaluating MDSC in metastatic melanoma patients comprising measuring the amount of MDSC having CD11b⁺ HLA-DR ^{-/low} CD14⁺ and "Lineage-FITC cocktail (CD3/CD16/CD19/CD20/CD56)" in Ficoll separated blood samples.

Meyer et al, 2014 discloses that the frequencies of circulating MDSC seemingly correlate with clinical outcome of melanoma patients treated with the therapeutic Ipilimumab comprising measuring the levels of Lin⁻ CD14⁺ HLA-DR⁻ monocytic MDSC levels.

Tarhini et al, 2014 discloses a method of determining the efficacy of treatment of melanoma patients receiving Ipilimumab including a step of assessing the level of MDSC of specific phenotypes in the patient and found that a greater decrease in circulating monocyte gate MDSC Lin1⁻ / HLA-DR⁻ /CD33⁺ / CD11b⁺ was seemingly associated with improved progression free survival (PFS).

Zhang et al, 2013 discloses that the presence of increased Lin ^{-/low} HLA-DR⁻CD11b⁺ CD33⁺ MDSCs in patients suffering from colorectal cancer is correlated with cancer stage and metastasis.

Lechner et al, 2011 discloses the functional characterization of two distinct human MDSC subsets, namely a) CD33⁺ HLA-DR^{low} HIF1alpha⁺ /STAT3⁺ and b) CD11b⁺ HLA-DR^{low} C/ EBPbeta⁺ by analyzing different human cancer cell lines of different cancer entities.

Idorn et al, 2014 found increased frequencies of CD14⁺ HLA-DR^{low/-} monocytic MDSCs ("M-DDSCs") which correlated with known negative prognostic markers in patients with prostate cancer including elevated levels of lactate dehydrogenase and prostate-specific antigen.

US2012/276004 discloses a method of diagnosing and monitoring the progression of cancer, cancer relapse, the efficacy of cancer treatment, or cancer staging of a cancer patient, in particular a lymphoma patient, including a step of assessing the level of MDSC of specific phenotypes, e.g. CD33⁺/HLA-DR^{low} or CD11b⁺/HLA-DR^{low} comprising phenotypes in the patient.

Chemotherapeutic drugs commonly used to treat cancer affect not only the tumor but also the immune system, having a crucial impact on anti-tumor responses and disease outcome (5, 9).

### GENERAL DESCRIPTION

In a first of its aspects, the present invention provides a method for predicting and/or monitoring a cancer patient's response to treatment with an immunotherapeutic agent, said method consisting of the steps of:
**(a)** Measuring the amount of myeloid derived suppressor cells (MDSC) having the profile CD11b⁺CD33⁺HLA-DR⁻ and/or CD11b⁺CD33⁺HLA-DR^{low}, in at least one biological sample being a whole blood sample obtained from the patient, wherein said whole blood sample is unfractionated and a frozen sample or a cryopreserved sample; and
**(b)** Determining whether the amount of MDSC of said profile is higher than a predetermined standard value or higher than a control sample;
wherein
detection of a high amount of MDSC of the above profile in the at least one biological sample as compared with the predetermined standard value or the control sample indicates that the patient is not responding and/or will not respond or is poorly responding and/or will poorly respond to treatment with said agent.

In one embodiment, said method is used for determining the patient's therapeutic regime by at least one of the following:
**(a)** Discontinuing treatment with said immunotherapeutic agent; or
**(b)** Combining the treatment with said immunotherapeutic agent with at least one additional compound being an anti-inflammatory and/or an anti-MDSC therapeutic agent.

In one embodiment, said measuring of the amount of myeloid derived suppressor cells (MDSC) having the profile CD11b⁺CD33⁺HLA-DR⁻ and/or CD11b⁺CD33⁺HLA-DR^{low} is performed prior to treatment with said therapeutic agent.

In other embodiments, said measuring of the amount of myeloid derived suppressor cells (MDSC) having the profile CD11b⁺CD33⁺HLA-DR⁻ and/or CD11b⁺CD33⁺HLA-DR^{low} is performed at least once, preferably more than once, during treatment with said therapeutic agent.

In one embodiment, said immunotherapeutic agent is selected from the group consisting of therapeutic antibodies, immune checkpoints inhibitors, cytokines, tumor infiltrating lymphocytes (TIL) and cancer vaccines.

In one specific embodiment, said immunotherapeutic agent is an anti CTLA4, anti PDL1 or anti PD1 agent.

In a specific embodiment said anti CTLA4 agent is the anti CTLA4 antibody Ipilimumab.

In another specific embodiment, said anti PD1 agent is the anti PD1 antibody lambrolizumab.

In certain embodiments, the cancer is selected from the group consisting of colorectal carcinoma or melanoma.

In one specific embodiment, the cancer is melanoma.

In one embodiment said melanoma is metastatic melanoma.

In another specific embodiment, the cancer is colorectal carcinoma.

In one embodiment, said colorectal carcinoma is metastatic colorectal carcinoma.

In certain embodiments said measuring of the amount of myeloid derived suppressor cells (MDSC) having the profile CD11b⁺CD33⁺HLA-DR- and/or CD11b⁺CD33⁺HLA-DR^{low} is performed by a method comprising the step of contacting detecting molecules specific for MDSC with the biological sample.

In certain embodiments, said detecting molecules are labeled detecting molecules. In other embodiments, said detecting molecules are attached to a substrate.

In one specific embodiment, said amino acid molecules are antibodies that specifically recognize and bind MDSC having the profile CD11b⁺CD33⁺HLA-DR⁻and/or CD11b⁺CD33⁺HLA-DR^{low}.

The present application describes that said antibody-bound MDSC are detected using fluorescence activated cell sorter (FACS), immunohistology, ELISA, RIA or Western blotting.

In certain embodiments, said method further comprises the step of at least one of:
**(a)** determining the expression levels of S100A8 and/or S100A9 proteins or encoding mRNA in said biological sample;
**(b)** determining the levels of cleaved caspase 3 in said biological sample;
**(c)** determining at least one of the level and/or activity of arginase 1 and iNOS in said biological sample;
**(d)** determining at least one of intracellular nitric oxide (NO) and reactive oxygen species (ROS) in said biological sample;
**(e)** determining the level of lactate dehydrogynase (LDH) in said biological sample;
**(f)** determining the level of MDSC suppressive activity on T cells in said biological sample.

The present application e.g. describes, that said MDSC suppressive activity on T cells is measured by assessing down regulation of CD247 and/or SNX9 expression, and/or impaired T cell proliferation.

In another aspect, the present invention provides a method for selecting a melanoma patient suitable for receiving treatment with an immunotherapeutic agent, said method consisting of the steps of:
**(a)** Measuring the amount of myeloid derived suppressor cells (MDSC) having the profile CD11b⁺CD33⁺HLA-DR⁻ and/or CD11b⁺CD33⁺HLA-DR^{low}, in at least one biological sample being a whole blood sample obtained from the patient, wherein said whole blood sample is unfractionated and a frozen sample or a cryopreserved sample; and
**(b)** Determining whether the amount of MDSC is lower than a predetermined standard value or lower than a control sample;
   wherein
   **(i)** said therapeutic agent is Ipilimumab or lambrolizumab or a combination thereof, and wherein
   **(ii)** detection of a low amount of MDSC in the at least one biological sample as compared with the predetermined standard value or the control sample indicated that the patient is suitable for receiving treatment with Ipilimumab or lambrolizumab or a combination thereof.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

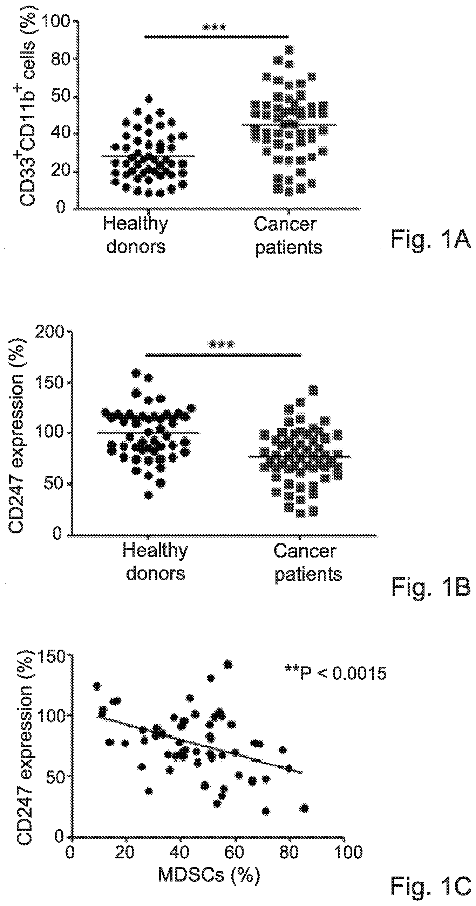

**Fig. 1A** is a schematic representation showing the percentage of CD33⁺ CD11b⁺ HLA-DR⁻ cells in peripheral blood obtained from healthy donors and from cancer patients (gated on HLA-DR^{-/-}cells). **Fig. 1B** is a schematic representation showing the CD247 expression presented as percent of mean fluorescence intensity (%MFI), in cells of healthy donors and cancer patients, gating was set on CD3⁺ cells. Fig. 1C is a schematic representation showing the correlation between MDSCs percentages and CD247 expression (shown as %MFI) in cancer patients; **P<0.0015; ***P<0.0001.

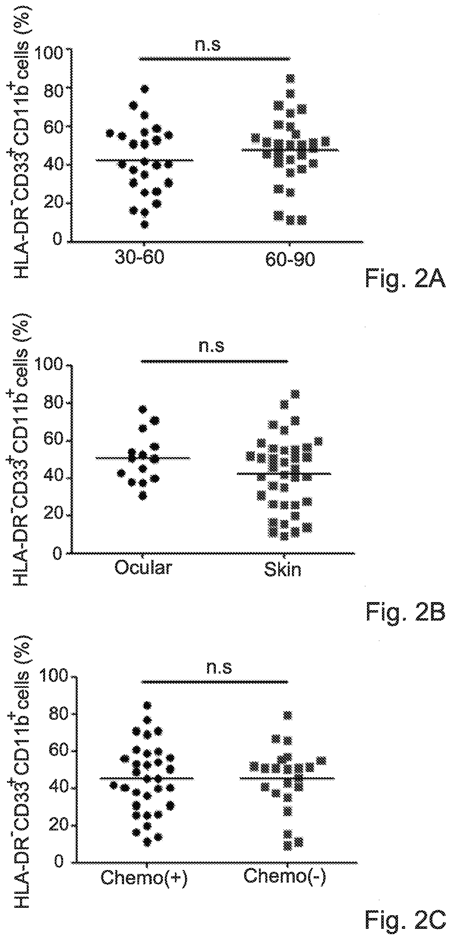

**Fig. 2A** is a schematic representation showing the percentage of CD33⁺ CD11b⁺ HLA-DR⁻ cells in the peripheral blood of two melanoma patient age groups: 30-60 years old and 60-90 years old. **Fig. 2B** is a schematic representation showing the percentage of CD33⁺ CD11b⁺ HLA-DR⁻ cells in the peripheral blood of two melanoma patient groups divided according to the tumor origin (skin or ocular). **Fig. 2C** is a schematic representation showing the percentage of CD33⁺ CD11b⁺ HLA-DR⁻ cells in the peripheral blood of two melanoma patient groups, one group received chemotherapeutic treatments (Chemo(+)) prior to ipilimumab treatments, and one group did not receive chemotherapeutic treatments (Chemo(-)) prior to ipilimumab treatments; n.s - not significant.

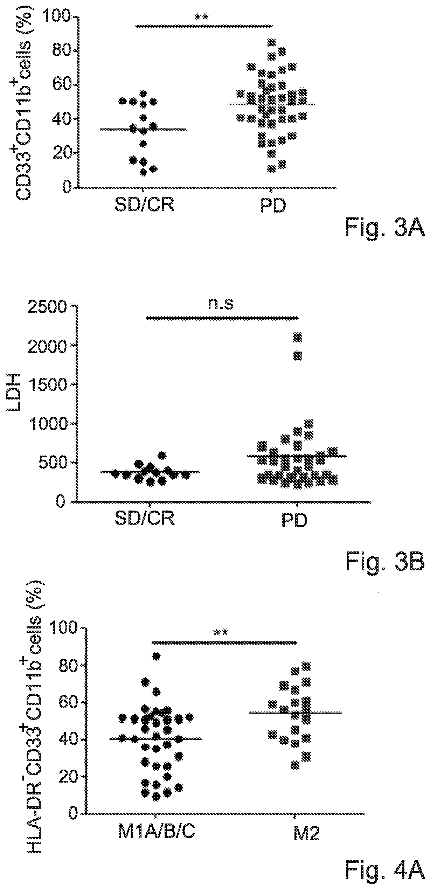

**Fig. 3A** is a schematic representation showing the percentage of CD33⁺ CD11b⁺ HLA-DR⁻ cells in the peripheral blood of two groups of melanoma patients: patients that responded to ipilimumab treatments (SD/CR; SD- stable disease, CR- complete response) and patients that did not respond to ipilimumab treatments (PD- progressive disease) (gated on HLA-DR^{-/-}cells). **Fig. 3B** is a schematic representation showing the lactate dehydrogenase (LDH) levels in the peripheral blood of patients that responded to ipilimumab treatments (SD/CR) and patients that did not respond to ipilimumab treatments (PD); **P<0.008, n.s - not significant.

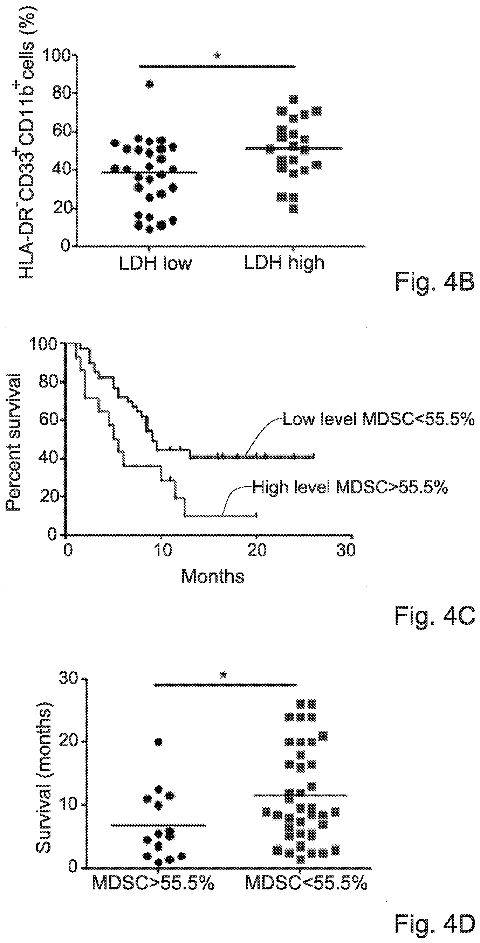

**Fig. 4A** is a schematic representation showing the percentage of CD33⁺ CD11b⁺ HLA-DR⁻ cells in patients with metastatic severity defined as MIA, M1B, and M1C (M1A/B/C) and in patients with higher metastatic severity defined as M2. Fig. 4B is a schematic representation showing the percentage of CD33⁺ CD11b⁺ HLA-DR⁻ cells in patients with low LDH levels and with high LDH levels (LDH low<480 U/I ; LDH high>480 U/I). **Fig. 4C** is a Kaplan-Meyer curve showing the percent survival in months of melanoma patients with Low level MDSC (<55.5%, n=39) and with High level MDSC (>55.5%, n=14). **Fig. 4D** is a schematic representation showing survival in months of patients in the Low level MDSC group (11,56±1.2, n=39) and the high level MDSC group (6.857±1.4, n=14); **P<0.008; *P<0.02.

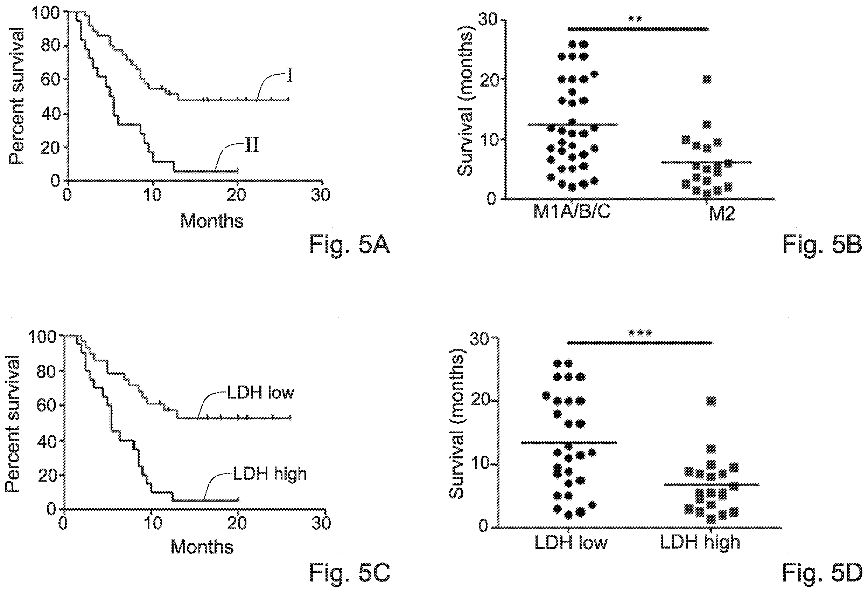

**Fig. 5A** is a Kaplan-Meyer curve showing the percent survival in months of two groups of melanoma patients (I and II) divided according to their metastatic level prior to ipilimumab treatments; I-M1A/B/C; II-M2. **Fig. 5B** is a schematic representation showing survival in months of M1A/B/C group (12.4±1.2, n=35) and M2 group (6.1±1.1, n=18). **Fig. 5C** is a Kaplan-Meyer curve showing the percent survival in months of two groups of melanoma patients divided according to their LDH levels prior to ipilimumab treatments; LDH low<480 U/I; LDH high>480 U/I. **Fig. 5D** is a schematic representation showing survival in months of LDH low group (13.5±1.4; n=28) and LDH high group (6.6±0.9, n=20); ***P<0.0009; **P<0.0021.

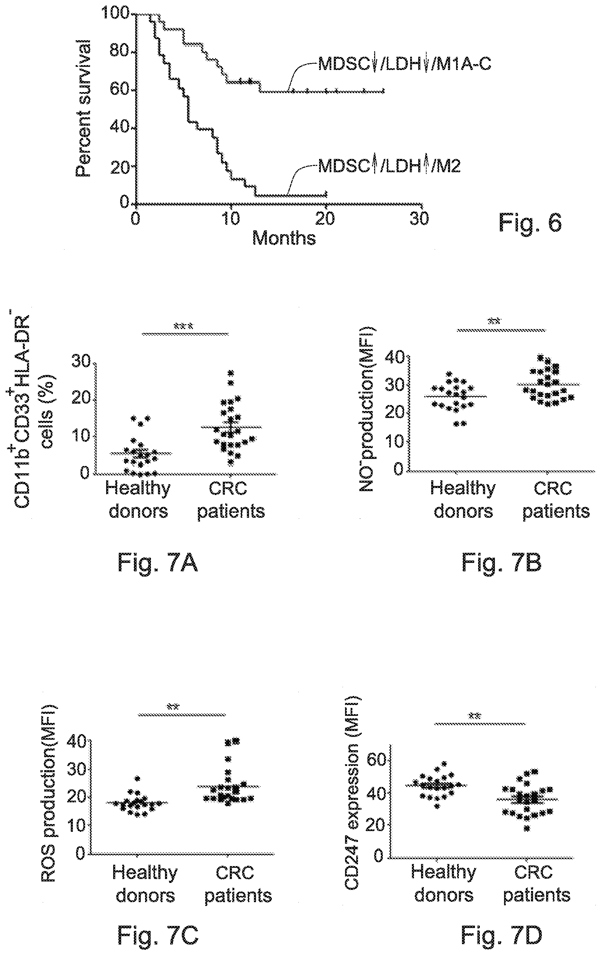

**Fig. 6** is a Kaplan-Meyer curve showing the percent survival in months of two groups of melanoma patients divided according to their initial parameters measured before the first ipilimumab treatment. One group had low MDSCs levels, low LDH levels and a M1A-C staging (MDSC↓LDH↓M1A-C); the second group had high MDSCs levels, high LDH levels and an M2 staging (MDSC↑LDH↑M2); P<0.0001.

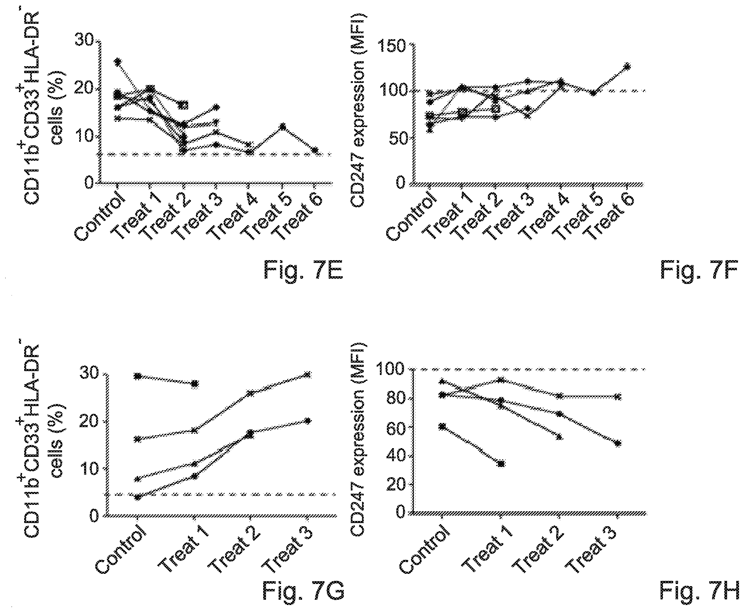

**Fig. 7A** is a schematic representation showing the percentage of CD11b⁺ CD33⁺ HLA-DR⁻ MDSC in 23 CRC patients and in 20 healthy donors. **Fig. 7B** is a schematic representation showing NO⁻ levels produced by MDSCs from healthy donors and CRC-patients presented as MFI, gating on CD11b⁺CD33⁺HLA-DR⁻ cells. **Fig. 7C** is a schematic representation showing ROS levels produced by MDSCs from healthy donors and CRC-patients presented as MFI, gating on CD11b⁺CD33⁺HLA-DR⁻ cells. **Fig. 7D** is a schematic representation showing CD247 expression presented as MFI, gating on CD3⁺ cells. **Fig. 7E** is a schematic representation showing the percentage of circulating CD11b⁺CD33⁺HLA-DR⁻ cells in CRC-patients before (control) and after 5-fluoruracil (5-FU/FOLFOX treatment in 6 patients. **Fig. 7F** is a schematic representation showing the expression of CD247 in T-cells in CRC-patients before (control) and after 5FU/FOLFOX treatment in 6 patients. **Fig. 7G** is a schematic representation showing the percentage of circulating CD11b⁺CD33⁺HLA-DR⁻ cells in CRC-patients before (control) and after FOLFIRI treatment in 4 patients. **Fig. 7H** is a schematic representation showing the expression of CD247 in T-cells in CRC-patients before (control) and after FOLFIRI treatment in 4 patients. Dashed lines represent the normal mean values of %MDSCs (Figs 7E and 7G) and CD247 levels (Figs 7F and 7H) that were measured in gated CD3⁺ cells and are presented as the expression in the experimental group relative to the mean of expression in healthy donors (as 100%);**, P<0.01; ***, P<0.001.

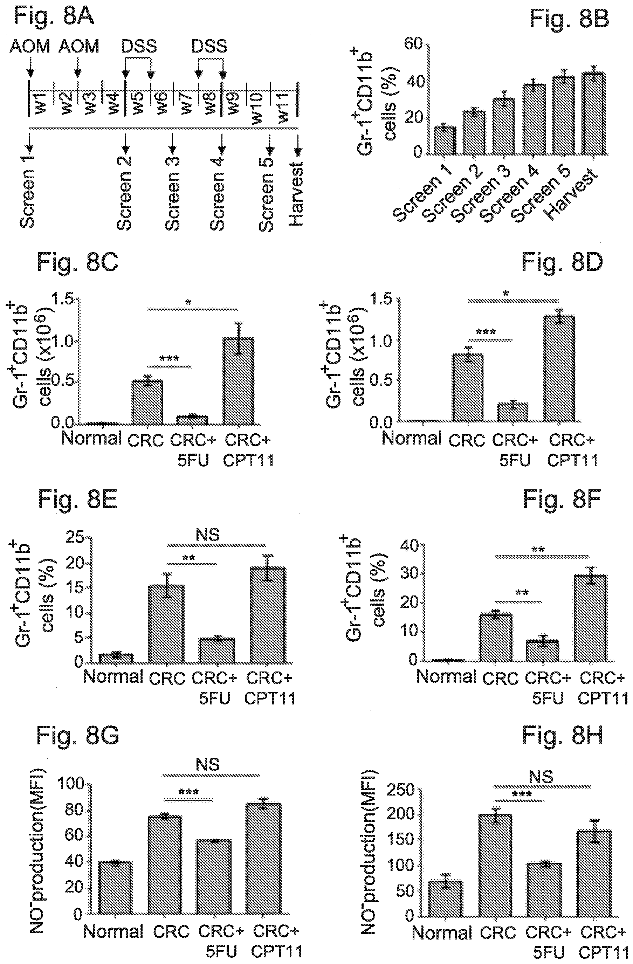

Fig. 8A is a schematic representation of the mouse model for CRC. Fig. 8B is a schematic representation of a kinetic study of Gr1⁺CD11b⁺ (mice MDSC) accumulation. Blood samples were collected from mice during CRC development and progression at the indicated time points, and tested for MDSC accumulation by flow cytometry. Figs. 8C and 8D are schematic representations of MDSC accumulation within the colons as evaluated by flow cytometry analysis. The Graphs represent the absolute number of MDSCs within the lamina propria (Fig. 8C) and the epithelium (Fig. 8D); *, P<0.05; ***, P<0.001; ns = non-significant. **Figs. 8E and 8F** are schematic representations of the levels of MDSC isolated from the lamina propria **(****Fig. 8E****)** and epithelium **(****Fig. 8F****)** of the colons from each experimental group as analyzed by flow cytometry. The graphs (means of triplicates ± s.e.m., n=4) are representative of a typical experiment out of three independent performed;**, *P*<0.01; ns = non-significant. **Figs. 8G and 8H** are schematic representations of NO⁻ production levels. The lamina propria **(****Fig. 8G****)** and epithelium **(****Fig. 8H****)** fractions isolated from the colons of CRC-mice were analyzed for NO⁻production by flow cytometry analysis gaiting on the MDSC population. Graphs represent production levels, as shown by MFI. All *in vivo* experiments involved 6 mice per group and were repeated three times yielding similar results. Graphs (means of triplicates ± s.e.m., *n*=6) are representative of a typical experiment out of three performed. *, P<0.05; ***, *P*<0.001; ns = non-significant.

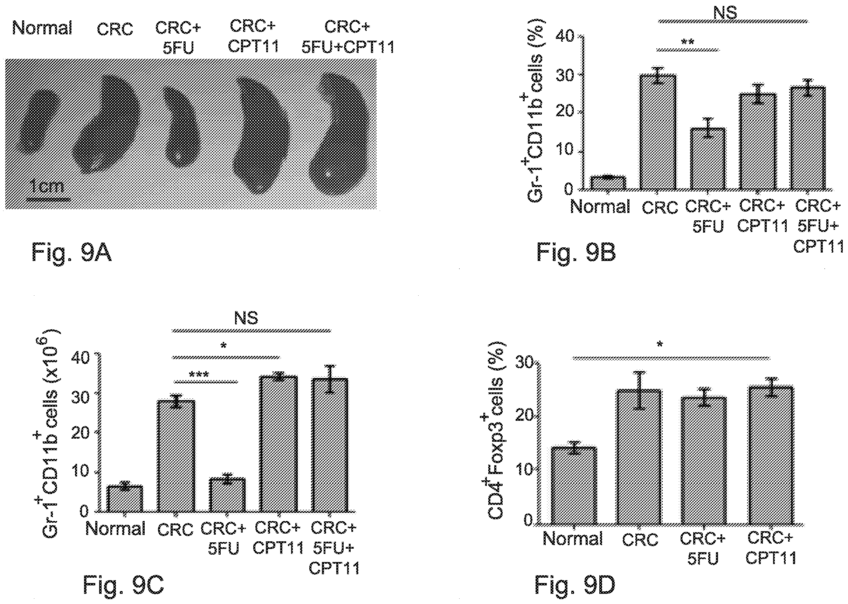

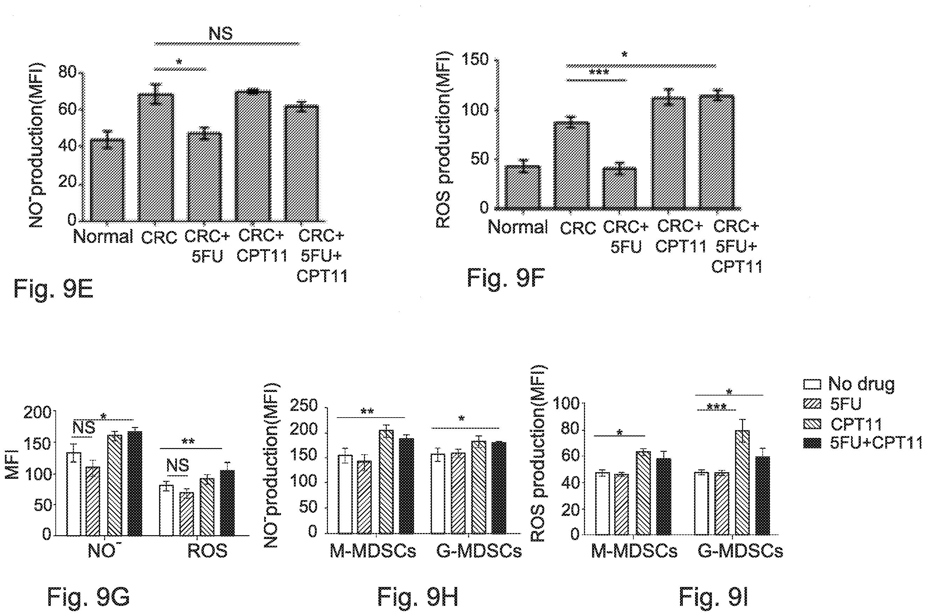

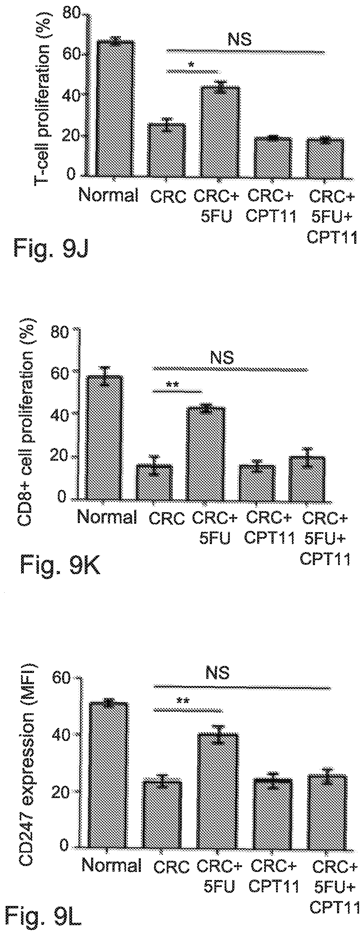

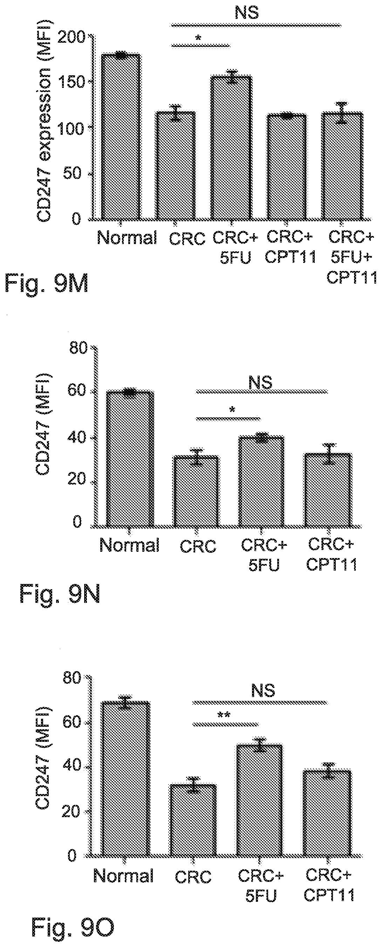

**Fig. 9A** is a photograph showing representative spleens of the different experimental groups. **Figs. 9B and 9C** are schematic representations of MDSCs accumulation as measured in the spleen by flow cytometry analysis, showing the percentage **(****Fig. 9B****)** and absolute numbers **(****Fig. 9C). Fig. 9D** is a schematic representation of the percentage of T regulatory cells in spleens from CRC-mice. Levels of Tregs from each experimental group were evaluated after fixation/permeabilization and double staining for CD4⁺Foxp3⁺. Graph (means of triplicates ± s.e.m., n=4) is representative of a typical experiment out of three performed. **Figs. 9E and 9F** are schematic representations of NO⁻ **(****Fig. 9E****)** and ROS **(****Fig. 9F****)** production. Splenocytes isolated from normal, CRC and 5FU-, irinotecan (CPT11)- and 5FU/CPT11-tretaed CRC-mice were analyzed for NO⁻ and ROS production by flow cytometry analysis, gating on MDSCs. Graphs represent mean fluorescence intensity (MFI). **Figs. 9G-9I** are schematic representations showing NO⁻ and ROS production in cultured MDSCs as analyzed by flow cytometry gaiting on CD11b⁺Gr1⁺ MDSCs **(9G),** or CD11b⁺Ly6C^{high} Ly6G⁻monocytic MDSCs (M-MDSCs) and CD11b⁺Ly6C^{low}Ly6G⁺ granulocytic MDSCs (G-MDSCs) sub-populations **(9H and 91).** **Fig. 9J** is a schematic representation showing the percent of proliferating T cells compared to steady-state levels of non-activated cells in each group. **Fig. 9K** is a schematic representation showing CD8+ cell proliferation. Splenocytes were labeled with CFSE and activated with anti-CD3 and anti-CD28 antibodies or left non-activated. The proliferative response was assessed by monitoring cell divisions of gated CFSE-labeled CD8⁺ T-cells. The percent of proliferating cells was calculated and compared to steady-state levels of non-activated cells in each group. **Fig 9L** is a schematic representation showing the expression level of CD247. Splenocytes from the experimental groups were analyzed for CD247 expression levels indicated by MFI, gating on CD3⁺ cells and. All *in vivo* experiments involved 6 mice per group and were repeated three times yielding similar results. Graphs (means of triplicates ± s.e.m., *n*=6) are representative of a typical experiment out of three performed. **Fig 9M** is a schematic representation showing the expression level of CD247. Splenocytes from the experimental groups were analyzed for CD247 expression levels gating on CD8⁺ cells and indicated by MFI. Graphs (means of triplicates ± s.e.m., n=5) are representative of a typical experiment out of three independent performed. 5FU and CPT11 display opposite effects on MDSC accumulation and CD247 expression in the colons of CRC-mice. **Figs 9N and 9O** are schematic representations showing the expression level of CD247. Cells were isolated from the lamina propria **(****Fig. 9N****)** and epithelium **(****Fig 9O****)** of the colons from each experimental group and analyzed by flow cytometry for CD247 expression levels (MFI), gating on CD3⁺ cells. Graphs (means of triplicates ± s.e.m., n=4) are representative of a typical experiment out of three independent performed. *, *P*<0.05;**, *P*<0.01; ns = non-significant.

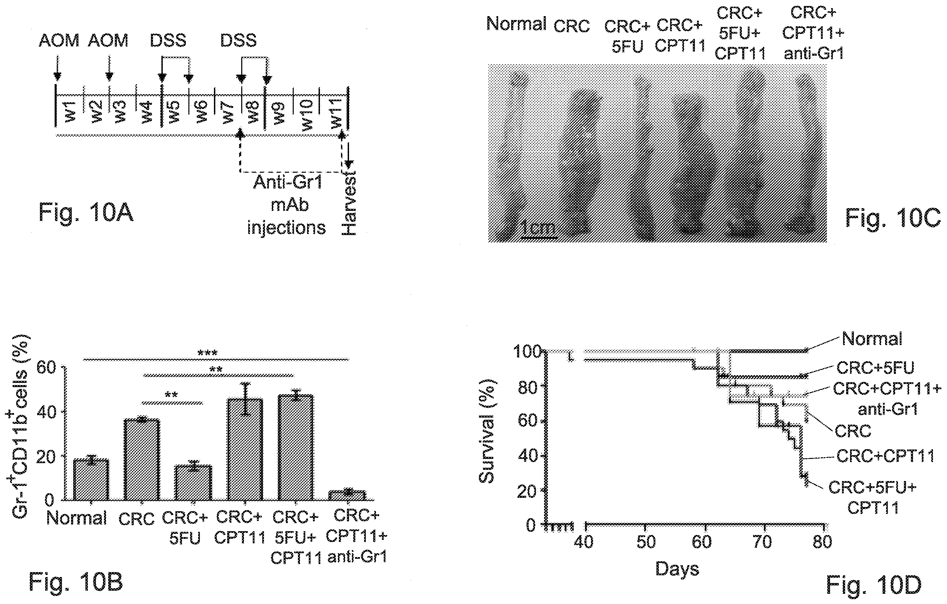

**Fig. 10A** is a schematic representation of the CRC mouse model including treatment timelines. Fig. **10B** is a schematic representation showing MDSCs accumulation as measured in PBLs by flow cytometry analysis. The graph represents the percent of MDSCs in each experimental group. **Fig. 10C** is a photograph showing representative colon structures. **Fig 10D** is a schematic representation showing Kaplan-Meyer curve (n = 20) of CRC, 5FU-treated, CPT11-treated, 5FU/CPT11-treated or MDSC-depleted CPT11-treated CRC-mice. All *in vivo* experiments involved 6 mice per group and were repeated three times yielding similar results. Graphs (means of triplicates ± s.e.m., n=6) are representative of a typical experiment out of three performed; **, *P*<0.01; ***, *P*<0.001.

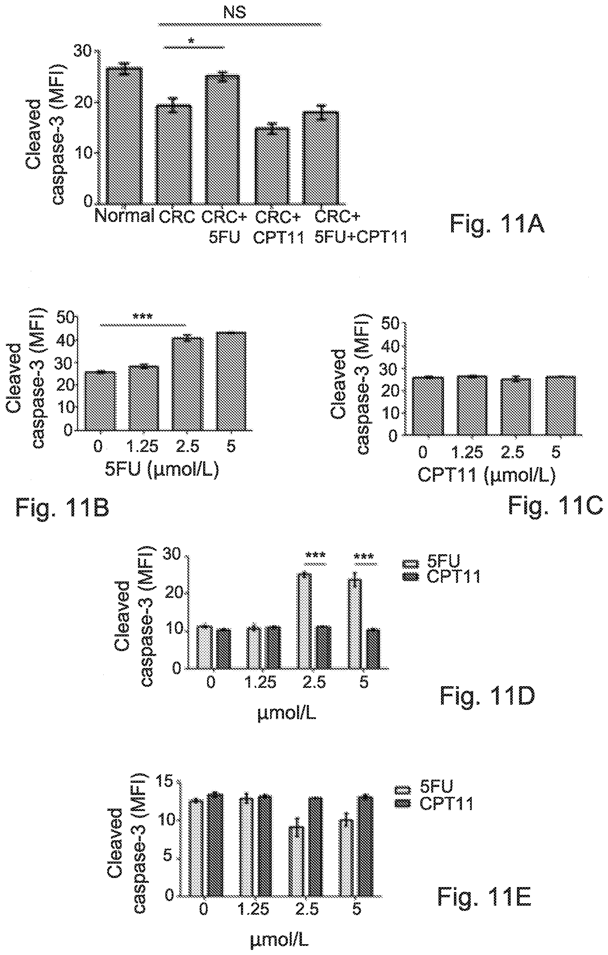

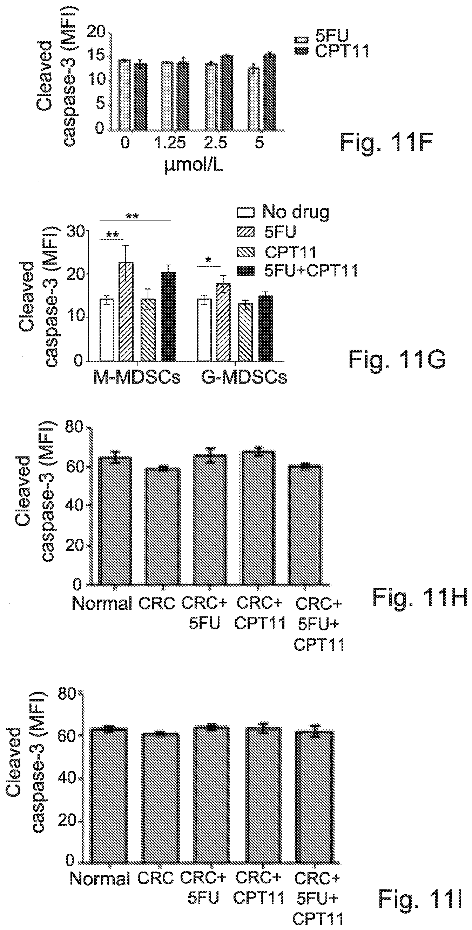

**Figs. 11A-I** are schematic representations showing levels of cleaved caspase 3. Splenic MDSCs from each group were analyzed for the expression of cleaved caspase-3 by flow cytometry analysis gating on MDSCs **(****Fig. 11A). Fig.11B** shows the effect of 5FU and **Fig. 11C** shows the effect of CPT11 on cleaved caspase-3 expression. **Fig 11D** shows the effect of the drugs on primary MDSCs. **Fig 11E** shows the effect of the drugs on differentiated CD11c⁺CD11b⁺DCs. **Fig 11F** shows the effect of the drugs on F4/80⁺CD11b⁺ macrophages. All experiments involved 6 mice per group and were repeated three times yielding similar results. Graphs (means of triplicates ± s.e.m.,*n*=6) are representative of a typical experiment out of three performed. *, P<0.05; ***, P<0.001; ns = non-significant. **Fig 11G** shows cleaved caspase-3 expression (MFI) in cultured MDSCs using flow cytometry analysis gaiting on CD11b⁺Ly6C^{high} Ly6G⁻monocytic MDSCs (M-MDSCs) and CD11b⁺Ly6C^{low}Ly6G⁺ granulocytic MDSCs (G-MDSCs) sub-populations. **Figs 11H** and **11I** show cleaved caspase-3 expression (MFI) in T (CD3⁺) lymphocytes **(****Fig 11H****)** and in B (B220⁺) lymphocytes **(****Fig 11I****).** All *ex vivo* experiments involved 5 and *in vivo* experiments 6 mice per group and were repeated three times yielding similar results. Graphs (means of triplicates ± s.e.m., n=6) are representative of a typical experiment out of three performed.

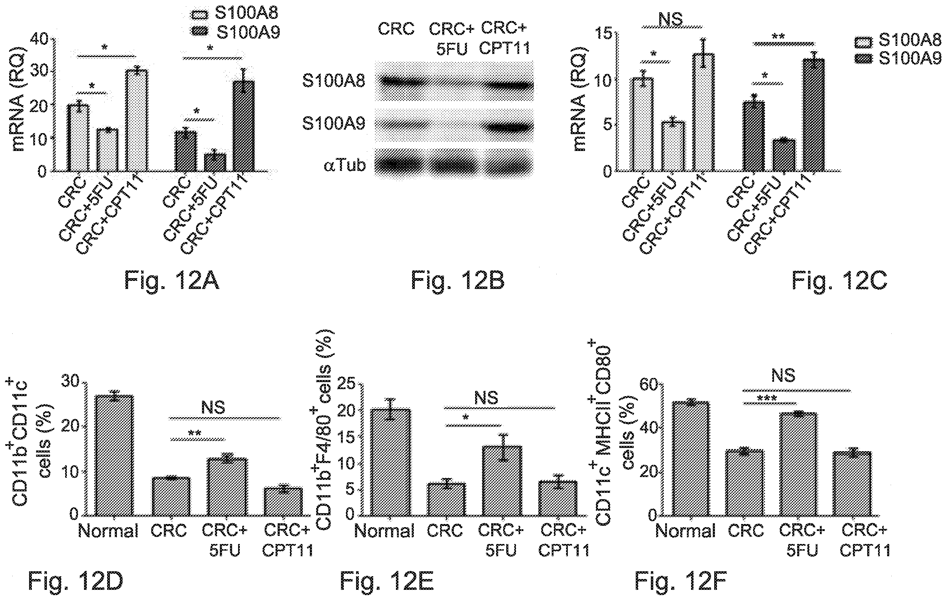

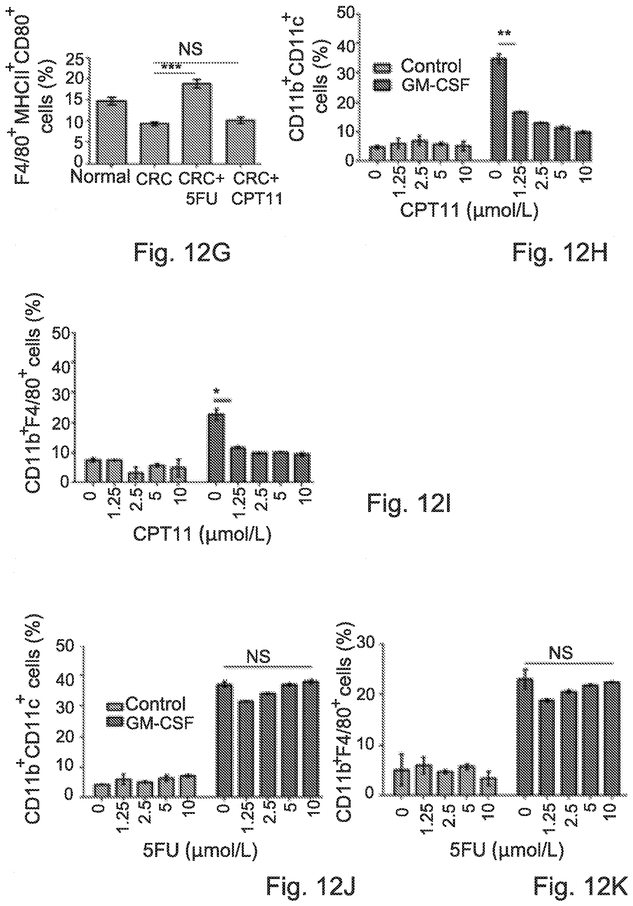

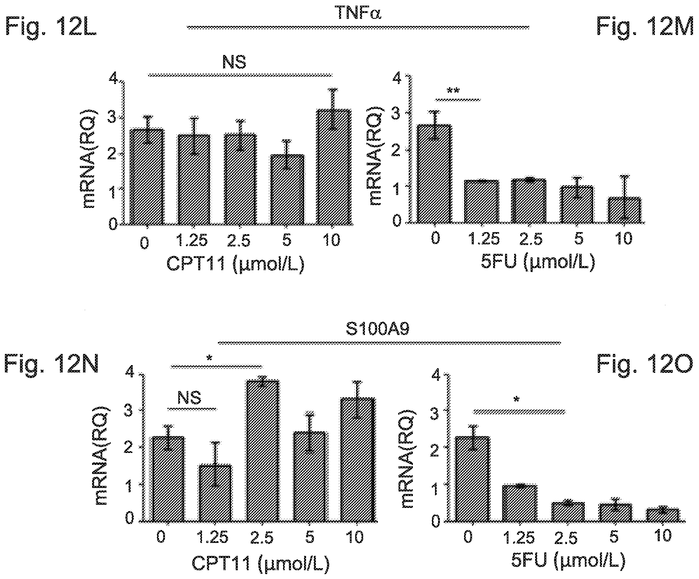

**Fig. 12A** is a schematic representation showing S100A8/9 mRNA levels and **Fig. 12B** is a schematic representation showing S100A8/9 protein levels in MDSCs isolated from the spleen of CRC-mice, or CRC-mice treated with 5FU or CPT11. **Fig 12C** is a schematic representation showing mRNA levels of S100A8/9 in the colon of CRC-mice treated with 5FU or CPT11 relative to the expression in untreated CRC-mice as evaluated by Real Time PCR analysis. **Figs. 12D-12G** are schematic representations of the levels of various cell populations: CD11c⁺CD11b⁺DCs **(****Fig. 12D****),** F4/80⁺CD11b⁺ macrophages **(****Fig. 12E****),** CD11c+MHCII+CD80+ cells **(****Fig. 12F****)** and F4/80⁺MHCII+CD80+ cells **(****Fig. 12G). Figs. 12H-12K** are schematic representations of MDSCs isolated from spleens of CRC mice that were *ex vivo* cultured with 10ng/ml GM-CSF in the absence or presence of scaled-doses (0, 1.25, 2.5, 5 and 10µmol/L) of CPT11 (Fig. 12H and 12I) or 5FU (Fig. 12J and 12K) for 3 days. The phenotype of differentiated DCs (Fig. 12H and 12J) and macrophages (Fig. 12I and 12K) was then evaluated. All *in vivo* experiments involved 6 mice per group and were repeated three times yielding similar results. Graphs (means of triplicates ± s.e.m., n=6) are representative of a typical experiment out of three independent performed. *Ex vivo* experiments involved 4 mice per group and were repeated three times yielding similar results. Graphs (means of triplicates ± s.e.m., n=4) are representative of a typical experiment out of three performed. Data shown are the mean ± s.e.m. *, P<0.05; **, P<0.01; ***, P<0.001 analyzed using 2-way ANOVA. Figs. 12L-12O are schematic representations of mRNA levels of various pro-inflammatory molecules generated by MDSCs. MDSCs isolated from spleens of CRC-mice (n=4) were cultured *ex vivo* in the presence of scaled-doses (0, 1.25, 2.5, 5 and 10µmol/L) of 5FU **(****Figs. 12M and 12O****)** or CPT11 **(****Figs. 12L and 12N****)** for 3 days. TNFα **(****Figs. 12** **L and 12M)** and S100A9 **(****Figs. 12N and 12O****)** mRNA levels were evaluated by Real Time PCR analysis performed on the primary MDSCs. Graphs (means of triplicates ± s.e.m., n=3) are representative of a typical experiment out of three independent performed; *, P<0.05; **, *P*<0.01.

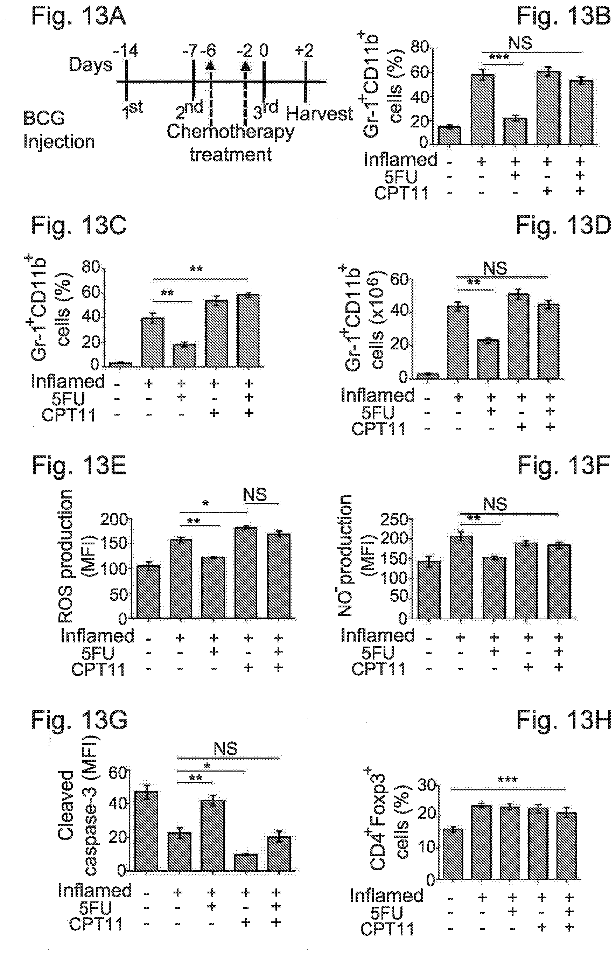

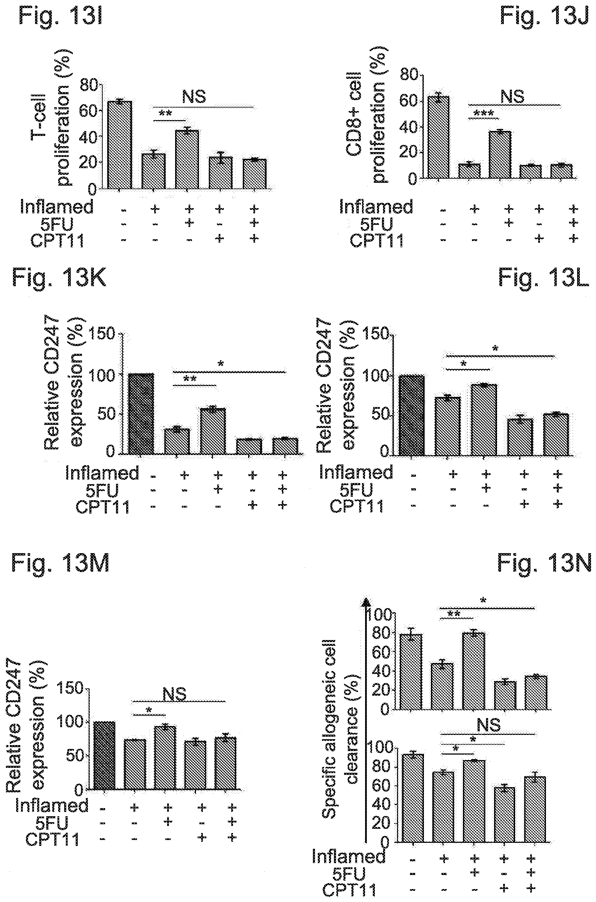

**Fig. 13A** is a schematic representation showing a mouse model for chronic inflammation. **Figs 13B and 13C** are schematic representations of the percentage of MDSCs within the PBLs **(****Fig 13B****)** and the spleen **(****Fig 13C****).** PBLs and spleens from normal, inflamed, inflamed 5FU-treated, CPT11-treated or 5FU/CPT11-treated mice were analyzed for MDSC accumulation by flow cytometry analysis. **Fig. 13D** is a schematic representation of the absolute number of MDSCs within the spleen of normal, inflamed and inflamed mice treated with 5FU, CPT11 or 5FU/CPT11. **Figs. 13E and 13F** are schematic representations showing NO⁻ **(****Fig. 13E****)** and ROS **(****Fig. 13F****)** production in splenocytes by flow cytometry analysis gating on the MDSC population. Graphs represent production levels, as shown by MFI. **Fig. 13G** is a schematic representation of cleaved caspase-3 levels. The expression of cleaved caspase-3 was analyzed by flow cytometry, gating on MDSC populations. **Fig. 13H** is a schematic representation of the percentage of Tregs. Tregs derived from the spleens of each experimental group were evaluated by measuring CD4⁺Foxp3⁺cells. **Fig. 13I** is a schematic representation of the percent of T cell proliferation. Splenocytes were labeled with CFSE and activated with anti-CD3 and anti-CD28 antibodies or left non-activated. The proliferative response was assessed by monitoring cell divisions of gated CFSE-labeled Thy1.2⁺ (CD90⁺) T-cells. The percent of proliferating cells was calculated and compared to steady-state levels of non-activated cells in each group. Fig **13J** is schematic representation showing percent CD8+ proliferation of splenocytes. **Figs. 13K-13M** are schematic representations showing relative CD247 expression. PBLs from each experimental group were double stained for CD247 **(****Fig. 13K****)** and CD3ε-chain **(****Fig. 13L****).** CD247 expression levels were measured in gated CD3⁺ cells and are presented as the expression in the experimental group relative to normal mice (as 100%). Splenocytes from each group were double stained for CD247 and NCR1. CD247 expression levels were measured in NK (NCR1⁺) cells and are presented as the expression in the experimental group relative to normal mice (as 100%) **(****Fig. 13M). Fig. 13N** is a schematic representation of the percent of specific allogeneic cell clearance. NK-cell mediated clearance of CFSE-labeled allogeneic (CFSE^{low}) and syngeneic (CFSE^{high}) splenocytes was evaluated by monitoring the ratio between CFSE^{low}/CFSE^{high} in the spleen (top) and PBLs (bottom) in each experimental group. The graphs of Figs. 13A-13C, 13E-13G, 13I, and 13N represent means of triplicates ± s.e.m., n=5. The graphs of Figs. 13D, 13H and 13K-13M represent means of triplicates ± s.e.m., n=4). All graphs are representative of a typical experiment out of three independent performed; *, *P*<0.05; **, *P<0.01;* ***, *P*<0.001; n.s. = non-significant.

### DETAILED DESCRIPTION OF EMBODIMENTS

The present invention is based on the surprising finding that the levels of myeloid derived suppressor cells (MDSC) having the profile CD11b⁺CD33⁺HLA-DR⁻ and/or CD11b⁺CD33⁺HLA-DR^{low} in a cancer patient's sample, as measured prior to commencing therapy, can serve as a predictor for the patient's response to therapy.

The present invention therefore provides a combinatorial analysis to evaluate the immune status of cancer patients prior to and following a given therapy, measuring MDSC levels or MDSC levels and their suppressive characteristics. Moreover, testing the patient prior to treatment will depict predictive criteria of whether the patient will respond to the therapy. As will be shown in Example 1 below, based on data on melanoma patients subjected to Ipilimumab treatment, it was shown that if high levels of MDSCs (more than 55%) are detected in the blood, this indicates immunosuppression and predicts, with very high significance, non-responsiveness to treatment and very low survival. Testing MDSCs prior to the given therapy also provides a tool to suggest pre or combined treatment with additional drugs that neutralize the inflammatory and immunosuppressive environment prior to or in conjunction with a given chemo or immune based therapy. This analysis is also performed in different intervals during the therapy to monitor changes in the immune status during treatment to evaluate whether the immune system functions properly; if an increase in MDSC levels (or the levels of their suppressive characteristics) is detected during treatment this indicates that the response to treatment may be reduced and hence the type of treatment, or its continuation can be reconsidered.

Chemotherapeutic treatments, and in particular immunotherapeutic treatments are costly and their success rates are currently poor, partly due to the fact that evaluations of the patients' immune status are not performed prior to and during treatment.

As demonstrated in Example 1 below, showing a clinical experiment analyzing 56 patients with melanoma (stage 4) subjected to Ipilimumab treatment, a significant correlation was found between high levels of MDSCs and low responsiveness to Ipilimumab treatment as reflected by poor survival.

The predictive value of MDSC measurements was also demonstrated in colorectal cancer as shown in Example 2 below. Colorectal cancer (CRC) is associated with chronic inflammation and immunosuppression mediated by myeloid-derived suppressor cells (MDSC). Although chemotherapy reduces tumor burden at early stages, it tends to have limited effect on progressive disease, possibly due to adverse effects on the immune system in dictating disease outcome. As shown in Example 2 advanced CRC patients display enhanced MDSC levels and reduced CD247 expression.

Monitoring the immune status of stage IV CRC patients, prior to and following FOLFOX or FOLFIRI treatments revealed that prior to therapy the patients displayed a suppressed immune status as indicated by the elevated MDSC levels and down-regulated CD247, which is a key molecule that "senses" immune functionality and regulates T- and NK-cell immune responses (10). Recent data demonstrated that 5FU treatment leads to a selective MDSC apoptosis and tumor regression in mice (11).

During chemotherapeutic treatments, while FOLFOX reduced accumulation of circulating MDSCs that was accompanied by up-regulated CD247 expression, FOLFIRI displayed opposite effects, enhancing the suppressive environment.

To gain better understanding of 5-fluoruracil (5FU) and Irinotecan (CPT11) adverse effects on host immunity, a mouse CRC model that mimics the human disease was used (1). Similar to the patients, CRC-mice displayed an immunosuppressive status. As shown in Example 2, CPT11 but not 5FU increases immunosuppression by inducing MDSC insensitivity to apoptosis, arresting their differentiation and retaining their suppressive features. Moreover, 5FU/CPT11 combined treatment displays harmful effects, resulting in a dysfunctional immune response associated with cancer progression and short survival, showing that CPT11 antagonizes the anti-cancer activity of 5FU by exerting its detrimental immunoregulatory effects.

These results highlight the importance of developing therapeutic regimens that can target both the immune system and the tumor in developing improved personalized treatments for cancer.

As used in the specification and claims, the singular forms "a", "an" and "the" include plural references unless the context clearly dictates otherwise.

The methods disclosed by the present application are applicable to any type of cancer patient. Cancers in accordance with the invention include colorectal carcinoma and melanoma.

In a specific embodiment the cancer is melanoma.

In a specific embodiment, the present invention provides a method for predicting a melanoma patient's response to treatment with ipilimumab.

In another specific embodiment the cancer is colorectal carcinoma.

As used herein the term ***"treating"*** or ***"treatment"*** of a disease in a patient refers to administration of a ***"therapeutic agent"*** intended for preventing one or more of the disease symptoms, inhibiting disease development, stabilizing its progression, or ameliorating or delaying the appearance of one or more of its symptoms.

The ***"therapeutic agent"*** in the context of the present invention is an immunotherapeutic agent. Immunotherapeutic agents are compounds that are well known in the art and their selection depends on the cancer being treated. This selection is well within the skill of an attending doctor.

Information regarding cancer therapeutic agents and known therapeutic combinations can be found in the US National Cancer Institute's web site http://www.cancer.gov/ or in the US National Comprehensive Cancer Network's web site, http://www.nccn.org/.

Cancer patients are subjected to various types of immune-based therapies since most tumors display immunogenic features. The aim of such therapies is to increase the function of the patients' immune system by blocking inhibitory and/or apoptotic receptors expressed on immune cells such as T cells or by controlling inhibitory signaling pathways and also to boost the immune system by immunization protocols or transfer of 'educated' anti-tumor T lymphocytes. Examples for the first strategy are the Ipilimumab treatment (for example in melanoma), which is an anti-CTLA4 antibody that neutralizes the inhibitory stage of the T lymphocytes aiming at increasing the immune system functionality, the lambrolizumab treatment, which is an anti-PDl antibody and the MPDL3280A treatment which is an anti-PD-Ll (the ligand of PD1) antibody. PD1-PD-L1 interactions also provide negative/apoptotic signals that dampened response of the immune system against the tumor. Blocking the harmful effects of these inhibitory receptors is supposed to induce recovery of the patient's immune function. However, if there are additional key factors that suppress the immune system, which dominate the environment, the suggested treatment will not succeed. Examples for the second strategy include immunization protocols using autologous or heterologous tumor cells with or without dendritic cells, and boosting the patient's immune system by transferring activated 'educated' anti-cancer T cells (TILs) in the presence or absence of cytokines and/or reagents used in the first strategy (adoptive T cell transfer). Again, a suppressive environment generated in the patient during tumor development will inhibit efficient responses to such immune boosting therapies.

Therefore, suitable immunotherapeutic agents according to the invention include, but are not limited to immunomudulatory agents including for example, therapeutic antibodies, immune checkpoints inhibitors, cytokines (e.g. IL-2 or interferon α), T cell modulators, tumor infiltrating lymphocytes (TIL), and therapeutic or preventive cancer vaccines.

*"Therapeutic antibodies"* in the context of the present invention relate to different types of monoclonal antibodies that are used in cancer treatment. Such antibodies are directed against tumor markers or antigens and exert their activity either by inducing antibody-dependent cell cytotoxicity (ADCC), or by association with a toxic agent. Non limiting example of therapeutic antibodies include: Anti-CD52 (Alemtuzumab, Campath®), Anti-HER2/neu (erbB2) receptor (Trastuzumab, Herceptin®), anti-HERl/EGFR (Cetuximab, Panitumumab); Anti-VEGF-A (Bevacizumab); Anti-CD20 (Rituximab, Tositumomab, Ibritumomab); and Anti-CD33 (Gemtuzumab).

The therapeutic antibodies also include immune checkpoint blocking antibodies such as anti-CTLA4 (e.g. Ipilimumab), anti-PD-1 (e.g. lambrolizumab) and anti-PD-Ll (the ligand of PD1) (e.g. MPDL3280A).

*"Immune checkpoints inhibitors"* are compounds that interfere in pathways that regulate the immune status of a patient. The immune system depends on several checkpoints to avoid activity of the immune system on healthy cells. Tumor cells often take advantage of these checkpoints to escape detection by the immune system. Examples of immune checkpoints are CTLA-4 and PD-1.

In specific embodiments said immunotherapeutic agent is Ipilimumab, lambrolizumab or their combination.

**"*Ipilimumab*"** (also known as MDX-010, MDX-101 or Yervoy) relates to a humanized anti-CTLA4 monoclonal antibody. By targeting CTLA4, Ipilimumab neutralizes the inhibitory stage of cytotoxic T lymphocytes and thereby activates these cells of the immune system and allows them to successfully target and destroy cancer cells. Ipilimumab is presently used in the treatment of melanoma patients.

**"*lambrolizumab*"** relates to a humanized anti-PDl monoclonal antibody. *"Tumor infiltrating lymphocytes"* (TILs) are a type of white blood cells found in tumors. TILs are implicated in killing tumor cells, and the presence of lymphocytes in tumors is often associated with better clinical outcomes. TILs may be used as an adoptive cell transfer therapy to treat cancer. For example, autologous lymphocytes are isolated from patients' tumors and grown to very large numbers of cells *in vitro.* Prior to TIL treatment, patients are given nonmyeloablative chemotherapy to deplete native lymphocytes that can suppress tumor killing. Once lymphodepletion is completed, patients are then infused with TILs in combination with interleukin 2 (IL-2).

*"Cancer vaccines"* stimulate the immune system's ability to fight cancer. Preventive (or prophylactic) vaccines are intended to prevent cancer from developing in healthy subjects, while treatment (or therapeutic) vaccines are intended to treat an existing cancer. A non-limiting example of a cancer vaccine is the antigen presenting cells (APC)-based sipuleucel-T (Provenge®) for treating metastatic prostate cancer.

An *"effective amount"* of an immunotherapeutic agent or a combination thereof is an amount sufficient to effect beneficial or desired results, i.e. an amount sufficient to treat, ameliorate, alleviate, inhibit disease development, stabilize disease, prevent or reduce symptoms of cancer in a cancer patient. An effective amount can be administered in one or more administrations, applications or dosages. Such delivery is dependent on a number of variables including the time period for which the individual dosage unit is to be used, the bioavailability of the immunotherapeutic agent, the route of administration, etc. It is understood however that specific dose levels of the immunotherapeutic agents in accordance with this aspect of the invention for any particular patient depends upon a variety of factors including the activity of the specific compound employed, the route of administration, the age of the patient, its gender, body weight, general health, additional drugs that the patients receives etc. Determining the precise dosages is well within the physician's skill.

The immunotherapeutic agent can be administered by any route as known in the art, for example by oral administration or by parenteral administration including intramuscular, intravenous or subcutaneous administration. In some embodiments, when oral administration is employed the agent is administered using a convenient daily dosage regimen, and may be in the form of tablets, pills, capsules, semisolids, powders, sustained release formulations, solutions, suspensions, aerosols or any other appropriate composition.

The immunotherapeutic agent is preferably administered in a pharmaceutical composition that may further comprise suitable pharmaceutical grade excipients and carriers. Such excipients and carriers include, but are not limited to, starch, cellulose, talc, glucose, lactose, sucrose, gelatin, malt, glycerol, propylene glycol, water, ethanol, oils of various origins, sodium chloride, saline, aqueous dextrose, and the like.

In one embodiment the method of the invention is used for determining the patient's therapeutic regime by at least one of the following:
**(a)** Discontinuing treatment with said immunotherapeutic agent; or
**(b)** Combining the treatment with said immunotherapeutic agent with at least one additional compound being an anti-inflammatory and/or an anti-MDSC therapeutic agent.

As used herein an ***"anti-inflammatory agent"*** relates to a compound that reduces inflammation. Anti-inflammatory agents include, but are not limited to, NSAIDs (non-steroidal anti-inflammatory drugs) including broad-spectrum NSAIDs (which non-specifically inhibit both COX-1 and COX-2) e.g. aspirin, sulindac, ibuprofen, piroxicam, and COX-2 specific agents, such as celecoxib, corticosteroids (e.g. the glucocorticoids dexamethasone, hydrocortisone and prednisone), and antibodies directed against cytokines associated with inflammation, e.g. TNFα.

As used herein an **"*****anti-** MDSC therapeutic **agent"*** relates to a compound that inhibits MDSC. Such a compound may act by deactivation of MDSC, differentiation of MDSC into mature cells, blocking development of MDSC, or depletion of MDSC. Deactivation of MDSC may be achieved by using nitric oxide (NO) inhibitors, phosphodiesterase-5 (PDE-5) inhibitors, Nitro-aspirins, L-NAME (N (G)-Nitro-L-Arginine Methyl Ester), Arginase inhibitors, COX2 inhbitors, NOHA, ROS inhibitors (e.g. synthetic triterpenoids), MDSC migration inhibitors (e.g. anti glycan antibodies and CSF-1R inhibitors), histamine inhibitors or anti IL-17 antibodies. Differentiation of MDSC into mature cells can be achieved by using vitamins (e.g. ATRA (all trans retinoic acid), Vitamin A, vitaminD3, IL-12 or CpG. Blocking development of MDSC can be achieved by using bisphosphonates (e.g. zoledronic acid), or by modulating cell signaling using JAK2/STAT3 inhibitors, multi-kinase inhibitors or VEGF inhibitors. Depletion of MDSC can be achieved for example by using cytotoxic agents (e.g. gemcitabine, cisplatin, paclitaxel or 5-FU), HSP 90 inhibitors (e.g. 17-DMAG), IL-6R blockers or antibody drug conjugates (Wesolowsli et al., J. for Immuno Therapy of Cancer 2013 1:10). In addition, MDSC depletion can be achieved using antibodies directed against MDSC cell markers, for example anti CD33 antibodies. For research purposes MDSC depletion in mouse models can be achieved for example by using anti Gr1 antibodies.

As used herein the term *"therapeutic regimen"* or *"therapeutic regime"* relates to a regulated scheme of treatment. This scheme of treatment comprises for example administering a therapeutic agent to a patient at specific intervals, either alone or in combination with additional therapeutic agents or treatment modalities (e.g. irradiation). In the context of the present invention, an *"altered"* therapeutic regimen or *"altering"* the therapeutic regimen relates herein to a change in the schedule of treatment which is brought about by the acquired information on the immune status of the patient, as exemplified in the levels of MDSC in a patient's biological sample. The change in schedule of treatment may be a decision to refrain from treating the patient with a immunotherapeutic agent (e.g. Ipilimumab), or, in case that the patient is already being treated with a immunotherapeutic agent, the change may be a decision to cease treatment. Alternatively, an altered therapeutic regimen may relate to a decision to include at least one additional therapeutic agent in the therapeutic regimen, a therapeutic agent which is directed to enhancing the patient's immune system and may be an anti-inflammatory drug and/or a drug directed at the elimination of MDSC.

As used herein the terms *"response", "responsiveness", "responsive"* or *"responder"* to treatment with an immunotherapeutic agent refers to an improvement in at least one relevant clinical parameter as compared to an untreated subject diagnosed with cancer, or as compared to the clinical parameters of the same subject prior to said treatment.

The term *"therapeutic* ***failure*",** *"non responder", "non-responsive"* or *"not respond"* to treatment with an immunotherapeutic agent, refers to a treated cancer patient not experiencing an improvement in at least one of the clinical parameters. This term also encompasses a poor response to therapy which indicates a very low level of response which is not clinically significant or sufficient. For example, low responsiveness to an immunotherapeutic agent treatment may be reflected by poor survival.

The methods of the invention include the step of measuring the amount of myeloid-derived suppressor cells (MDSC) having the profile HLA DR⁻ CD33⁺ CD11b⁺ or HLA DR^{low} CD33⁺ CD11b⁺ in a biological sample obtained from the patient.

*"MDSC"* are a heterogeneous population of early myeloid progenitors, immature granulocytes, macrophages, and dendritic cells at different stages of differentiation. These cells have the capacity to suppress both the cytotoxic activities of natural killer (NK) and NKT cells, and the adaptive immune response mediated by CD4⁺ and CD8⁺ T cells. Human MDSCs commonly express Siglec-3/CD33 and lack lineage markers and HLA-DR, but heterogeneous expression of other cellular markers indicate that multiple subsets exist. Multiple positive markers used to identify MDSC are known in the art. These include, as non limiting examples, expression of CD33, CD14, CD15, CD66b, or CD11b.

The MDSC of the present invention are characterized as having the phenotype HLA DR⁻ CD33⁺ CD11b⁺ or HLA DR^{low} CD33⁺ CD11b⁺.

**"*HLA-DR*"** is a MHC class II cell surface receptor encoded by the human leukocyte antigen complex.

**"*CD11b*"** is expressed on the surface of many leukocytes including monocytes, granulocytes, macrophages, and natural killer cells.

*"CD33"* or Siglec-3 is a trans-membrane receptor expressed on cells of myeloid lineage. CD33 is usually considered myeloid-specific, but it can also be found on some lymphoid cells.

The presence of HLA DR⁻ CD33⁺ CD11b MDSC in the biological sample is determined using detecting agents which can be antibodies, specifically, anti HLA-DR antibodies, anti CD11b antibodies and anti CD33 antibodies. Wherein the presence or absence of each of the cell markers is denoted by a + or a - sign, respectively. Thereby HLA DR⁻ CD33⁺ CD11b MDSC are MDSC which lack HLA-DR, and express CD33 and CD11b. The designation HLA DR^{low} relates to MDSC which show a relatively low expression level of HLA-DR. When staining a cell population that comprises MDSC with anti-HLA DR antibodies, three cell populations can be identified according to the intensity of staining- HLA DR ^{high}, HLA DR^{low} and HLA DR^{negative}. The present invention relates to the cell populations that show negative and/or low staining with anti HLA-DR antibodies. The definitions of "high", "low" and "negative" staining levels are relative in each tested sample and are well within the knowledge of the skilled person in the art of the invention.

As indicated above, in certain embodiments the detecting agents can be antibodies. Antibodies may be prepared using methods well known in the art (see for example Harlow and Lane, Antibodies: A Laboratory Manual, Cold Spring Harbor Laboratory, New York (1988)).

The term ***"Antibody"*** as used herein refers to IgG, IgM, IgD, and IgA antibodies. This term refers to whole antibodies or fragments of the antibodies comprising the antigen-binding domain, e.g. scFv, Fab, F (ab') 2, bi-specific antibodies, diabodies, and other fragments capable of binding to the target molecule. The definition includes polyclonal antibodies and monoclonal antibodies. The monoclonal antibodies can be derived from various species such as murine, rabbit, goat or rat. Non limiting examples of commercial antibodies that can be used to identify the HLA DR⁻ CD33⁺ CD11b MDSC of the invention are provided in the Examples section below.

As used herein a ***"high level"*** or ***"higher amount"*** of MDSC detected in a patient's sample relates to a percent of cells that is significantly (e.g. as determined by statistical determination) higher than a predetermined standard value or significantly higher than a control sample. A ***"control sample"*** relates to a sample obtained from a healthy subject, i.e. a subject that does not suffer from a disease associated with chronic inflammation and is known to have a functional immune system. ***"Standard"*** or a "*predetermined **standard"*** as used herein, denotes either a single standard value or a plurality of standards with which the level (percent of) MDSC in the tested sample is compared. The standards may be prepared by determining the level of MDSC present in a sample obtained from a plurality of healthy subjects. After such standards are prepared, it is possible to compare the level of MDSC obtained from a specific tested subject to the corresponding value of the standards, and thus obtain an assaying tool.

More specifically, in certain embodiments, wherein "higher" or "high" levels of MDSC are indicated, it is meant that MDSC are present in between about 5% to 100%, more specifically about 10%, or about 20%, or about 30%, or about 40%, or about 50%, or about 60%, or about 70%, or about 80%, or about 90%, higher amounts than in the control sample or the predetermined standard.

Non limiting examples of higher levels of MDSC in patients' samples are demonstrated in the Examples below. In a specific embodiment a high level of MDSC in a sample relates to a percent value of above 50% MDSC per total cells in the sample, preferably above 55% of the cells in the sample.

As used herein the percent of MDSC relates to the percent of MDSC having the profile CD33⁺ CD11b⁺ within the HLA DR⁻ population of cells in the tested biological sample, or the percent of MDSC having the profile CD33⁺ CD11b⁺ within the HLA DR⁻and the HLA DR^{low} population of cells in the tested biological sample

The presence of a high level of MDSC in the cancer patient's sample indicates that the patient is immune suppressed and therefore predicts a poor response to treatment with the therapeutic agent, at least as a single agent. However, since the patient is identified as being immune suppressed the therapeutic regimen offered to the patient may be altered by including therein in addition to the cancer therapeutic agent, additional therapeutic agents capable of counteracting the detrimental effects of MDSC.

Additional parameters may be measured and combined with the level of MDSC in order to determine the patient's response to therapy.

The additional parameters include measuring the level of LDH (lactate dehydrogenase) prior to or during therapy, measuring the level of various suppressive characteristics of the patient and assessing the metastatic status of the patient.

"*LDH*" (lactate dehydrogenase) is an enzyme found in nearly all living cells. LDH catalyzes the conversion of pyruvate to lactate and back, as it converts NADH to NAD⁺ and back. Detection techniques for LDH are well known in the art, using for example enzymatic reactions performed on patients' serum samples or assays that are commercially available.

Relatively high levels of LDH (e.g. levels higher than 450 U/I, 480U/I, or 500 U/I, preferably higher than 480 U/I) are predictors of a shorter survival time of the cancer patient.

Similarly, a more advanced metastatic state of the patient is a predictor of a shorter survival time of the cancer patient.

As used herein ***"suppressive characteristics"*** of MDSC denote various measurable features or molecules that can be used as surrogates to determine the suppressive state of the MDSC. Such characteristics include, but are not limited to the expression levels of LDH, S100A8 and/or S100A9 proteins, the levels of cleaved caspase 3, intracellular nitric oxide (NO), reactive oxygen species (ROS), iNOS, and arginase 1, levels and activity in said biological sample; and suppressive activity of the MDSC on T cells. In certain embodiments, MDSC suppressive activity on T cells is measured by assessing down regulation of CD247 and/or SNX9 expression, and/or impaired T cell proliferation.

***"S100A8"*** is a calcium- and zinc-binding protein which plays a prominent role in the regulation of inflammatory processes and immune response. It can induce neutrophil chemotaxis and adhesion. S100A8 functions involve proinfammatory, antimicrobial, oxidant-scavenging and apoptosis-inducing activities.

***"S100A9"*** is a calcium-binding protein also known as migration inhibitory factor-related protein 14 (MRP14) or calgranulin B. It is encoded by the *S100A9gene.*

These proteins are predominantly found as calprotectin (a complex of S100A8/A9) which has a wide plethora of intra- and extracellular functions. Detection of S100A8 and/or S100A9 can be done using antibodies which are specific for these proteins. Such antibodies are commercially available for example from R&D Systems. mRNA encoding these proteins can be detected using appropriate probes, e.g. as shown in the Examples.

***"Caspase-3"*** (CASP3) is a caspase protein that interacts with caspase-8 and caspase-9. It is encoded by the *CASP3* gene. The **CASP3 protein** is a member of the **c**ysteine-**asp**artic acid prote**ase** (caspase) family. Sequential activation of caspases plays a central role in the execution-phase of cell apoptosis. Caspases exist as inactive proenzymes that undergo proteolytic processing at conserved aspartic residues to produce two subunits, large and small, that dimerize to form the active enzyme. This protein cleaves and activates caspases 6 and 7; and the protein itself is processed and activated by caspases 8, 9, and 10. Detection of the cleaved form of caspase 3 hence indicates that the cell is undergoing apoptosis. Detection of cleaved caspase 3 can be done using antibodies which are specific for the cleaved form. Such antibodies are commercially available for example from R&D Systems, sigmaAldrich etc.

***"Intracellular Nitric oxide"*** (NO) is an important cellular signaling molecule involved in many physiological and pathological processes. ***"Reactive oxygen species"*** (ROS) are chemically reactive molecules containing oxygen. Examples include oxygen ions and peroxides. ROS are formed as a natural byproduct of the normal metabolism of oxygen and have important roles in cell signaling and homeostasis. An increase in ROS levels may result in significant damage to cell structures.

Detection techniques for NO or ROS are well known in the art, using for example assays that are commercially available. For example, ROS may be detected using ROS Brite™ APF which is a fluorogenic probe to measure hydroxyly radical in cells using conventional fluorescence microscopy, high-content imaging, microplate fluorometry, or flow cytometry. The cell-permeant ROS Brite™ APF reagent is nonfluorescent and produces bright green fluorescence upon reaction with hydroxyl radical. The resulting fluorescence can be measured using fluorescence imaging, high-content imaging, microplate fluorometry, or flow cytometry. NO may be detected using a cell permeable analog of DAF-2 that is hydrolyzed to DAF-2 by intracellular esterases. This agent can be used in fluorescence microscopy to measure real-time changes in nitric oxide (NO) levels. Exemplary protocols for measuring NO and ROS are provided in the Examples.

***"iNOS"*** (inducible NO Synthase) is an enzyme catalyzing the production of NO from L-arginine and is a member of the Nitric oxide synthases family. It is an inducible NO synthase and is involved in immune response, binds calmodulin at physiologically relevant concentrations, and produces NO as an immune defense mechanism, as NO is a free radical with an unpaired electron. Detection of iNOS can be done for example using antibodies which are specific for this enzyme. Such antibodies are commercially available (e.g. by R&D Systems).

***"Arginase"*** (also termed arginine amidinase, canavanase, L-arginase, arginine transamidinase) is a manganese-containing enzyme. It is the final enzyme of the urea cycle. Detection of Arginase 1 can be done for example using antibodies which are specific for this enzyme. Such antibodies are commercially available (e.g. by SigmaAldrich).

*"CD247"* (Cluster of Differentiation 247) refers to T-cell surface glycoprotein CD3 zeta chain also known as T-cell receptor T3 zeta chain. Detection of CD247 on the surface of T cells can be done using antibodies which are specific for this marker. Such antibodies are commercially available. An example for CD247 detection technique is provided in the Examples.

The *"SNX9"* (Sorting nexin-9) protein is a member of the sorting nexin family. Members of this family contain a phox (PX) domain, which is a phosphoinositide binding domain, and are involved in intracellular trafficking. Detection of SNX9 in T cells can be done using antibodies which are specific for this marker. Such antibodies are commercially available. An example for SNX9 detection technique is provided in the Examples.

Elevated levels of NO, elevated levels of ROS, elevated levels of S100A8 and/or S100A9 proteins, low levels of cleaved caspase 3, and MDSC suppressive activity on T cells indicate that the patient suffers from an immune compromised state which affects or will affect the patient's responsiveness to therapy.

As used herein a ***"biological sample"*** refers to any cell containing sample obtained from the patient. The sample can be a peripheral blood sample including whole blood. The sample may be previously frozen or cryopreserved.

Methods for cryopreservation of cells and tissues are well known in the art, e.g. by mixing the cells with dimethyl sulfoxide (DMSO). One example of a cryopreservation protocol is provided in the Examples below.

The HLA DR⁻ CD33⁺ CD11b MDSC in the biological sample can be detected using fluorescence activated cell sorter (FACS), immunohistology (immunohistochemistry of tissue sections), as well as ELISA, RIA or Western blotting of the suitable MDSC cell markers i.e. CD33, CD11b. In addition, the MDSC in the biological sample can be detected using nucleic acid-based detection assays including in situ hybridization, RT-PCR (real-time polymerase chain reaction) and Northern blotting with probes specific for the MDSC markers, i.e. CD33, CD11b.

A first step in some of the detection methods requires staining of the cells with the relevant antibodies. Methods for staining cells with antibodies are well known in the art and usually the appropriate protocols are included in the instructions that accompany any commercially available antibody. An example of a staining procedure is provided in the Examples below. Briefly, the cells (e.g. in the whole blood sample) are placed in suitable plates, washed with staining buffer, incubated with the antibodies (preferably labeled antibodies), followed by additional washing. For intracellular markers, the cells are fixed prior to staining (e.g. with paraformaldehyde) and permeabilized (with a permeabilization buffer, comprising e.g. PB-0.1% saponin, and 1% human serum.

The detection of the HLA DR⁻ CD33⁺ CD11b MDSC in accordance with the invention may be performed by flow cytometry in a fluorescence activated cell sorter (FACS). Protocols for carrying out FACS analysis are well known in the art. FACS provides a method for analysis and sorting a heterogeneous mixture of cells in a biological sample, one cell at a time, based upon the specific light scattering and fluorescent characteristics of each cell. In general, a first step in flow cytometry is obtaining cells in suspension, labeling the cells with fluorescently labeled agents (preferably antibodies) which bind specifically the desired markers. The cell may be labeled as a whole living cell, or permeabilized prior to labeling (using a fixative) in order to allow labeling of intracellular molecules. The labeled cells are than subjected to flow analysis in the FACS. A non-limiting example of a protocol for carrying out a FACS analysis is provided in the Examples below.

The detection of the HLA DR⁻ CD33⁺ CD11b MDSC in accordance with the present application may be also performed by immunohistochemistry of tissue sections. Protocols for carrying out immunohistochemistry are well known in the art. In general, a first step in immunohistochemistry is the preparation of tissue sections. For example, a tissue biopsy potentially comprising the cells is fixed (e.g. using formalin or paraformaldehyde), the fixed biopsy is embedded in a paraffin block and thin tissue sections or slices are prepared (e.g. in a microtome). Alternatively, the tissue biopsy is frozen in liquid nitrogen and thin tissue sections or slices are prepared (e.g. using a cryostat). The tissue sections are than stained with labeled detecting agents (e.g. antibodies). The label in such cases may be enzymatic. A non-limiting example of a protocol for carrying out immunohistochemistry is provided in the Examples below.

Dot blot, slot blot, Western blot and ELISA analyses are commonly used assays for detecting the presence and amount of target molecules (usually proteins) in a sample. In a dot blot assay the whole sample which putatively includes the target molecule is placed as such onto a porous substance (e.g. a nitrocellulose membrane) and the proteins within the sample are immobilized in the membrane in the form of a dot.

In a Western blot assay, the sample which putatively includes the target molecule is first run on a separating gel thereby the proteins are separated one from the other and form a gradient according to their size. Next, the separated proteins are blotted onto a nitrocellulose membrane and immobilized onto the membrane according to their respective position in the gel.

In an ELISA (Enzyme-linked immunosorbent assay) the whole sample which putatively includes the target molecule is placed as such onto a non-porous substance (e.g. a well of a standard 96 well plate) and the proteins within the sample coat the bottom of the well. Alternatively, sandwich ELISA assay may be performed. In such case, the bottom of the well is pre-coated with specific primary antibodies directed against a target molecule. Hence the target molecule is immobilized onto the surface via specific binding to these antibodies. Next, the presence and amount of the target molecule is assessed by an immunoassay conventionally employing a first and a second antibody, whereby the second antibody is detectably labeled so as to detect the presence of the target molecule and its amount.

The detection in accordance with the invention is performed using detecting molecules. The term ***"detecting molecules"*** as used herein refers to any molecule that can specifically recognize the MDSC markers of the invention or any one of the suppressive molecules. The detecting molecules may be amino acid molecules or nucleic acid molecules. Non limiting examples of detecting molecules include antibodies, receptors, ligands, substrates or nucleic acid probes. The detecting molecules may be detectably labeled.

The term ***"detectably labeled"*** as used herein refers to a detecting molecule which is associated with a compound that may be detected by an appropriate reaction (enzymatic or color reaction) or by fluorescent excitation and assists in visualizing, quantifying or detecting the target molecule (the labeling agent).

The labeling agent may be a fluorescent compound, e.g. fluorescein (or a fluorescein derivative such as FITC) or phycoerythrin (PE), a fluorescent particle such as quantum dot, an enzyme such as horseradish peroxidase (HRP) or alkaline phosphatase (AP), a chromophore, or an electrochemically active or a radioactive molecule.

It should be emphasized that the detectably labeled detecting molecules are non-naturally occurring molecules.

The term *"Association"* or ***"associated"*** as used herein refers to any physical or chemical forces such as Van-der-Walls, coordinative, covalent, ionic, electrostatic, dipole-dipole, or hydrogen association (bond or interaction). The association may occur directly or indirectly (i.e. comprising one or more intermediate agents). In some embodiments the intermediate agent is another protein, ligand or spacer. For example, the intermediate agent may be streptavidin, biotin, immunoglobulin binding protein (e.g. protein A, protein G, Protein A/G, protein L, etc.), DNA or RNA molecule, peptide tag or its chelating complex (e.g. polyhistidine tag or metal complex of nitrilotriacetic acid such as Ni-NTA).

The detecting molecules in accordance with the present invention may also be immobilized on a substrate, e.g. a membrane, a filter, beads.

The substrate may be a porous substrate. As used herein, the term ***"porous substrate"*** refers to a substrate having a plurality of pores (depressions). These pores have inner voids of the same or varying volume and shape, defined by inner surface. In certain embodiments the substrate pores are nanometric in size, namely having a mean size smaller than 1,000 nm. In some embodiments, the mean pore size is below 500 nm. In other embodiments, the mean pore size is below 300 nm. In further embodiments, the mean pore size is below 200 nm. In other embodiments, the mean pore size is below 100 nm. In other embodiments, the mean pore size is below 50 nm. In further embodiments, the mean pore size is between 300 nm and 50 nm. In specific embodiments the porous substrate is a membrane having 200nm or 450nm pores.

The substrate may also be a non-porous substrate.

The substrate may be a flexible or a rigid or a soft substrate, and may be composed of any material. In some embodiments the substrate is a layered substrate, e.g., a porous layer on a non-porous layer or soft layer (such as gel or tissue) on metal layer (holder). The substrate (or one of its layers) may be composed of insulating, conducting or semiconducting material. In some embodiments the substrate may be composed of glassy, polymeric, ceramic, fibrous material, or any combination thereof. In some embodiments the substrate's material may be composed of glass, paper, wool, fleece, gel, cellulose, or any combination thereof. In some embodiments the substrate is composed of a nitrocellulose or PVDF membrane. The nitrocellulose or PVDF membrane may have varying pore sizes, e.g. 0.2 µm (200 nm) or 0.45 µm (450 nm).

As indicated above, in certain embodiments the method further comprises measuring the expression level of certain suppressive features or markers. These include, but are not limited to LDH, S100A8 and/or S100A9 proteins, cleaved caspase 3, intracellular nitric oxide (NO), reactive oxygen species (ROS), iNOS, and arginase 1, CD247 and/or SNX9. The expression levels of the above noted suppressive markers in the biological sample can be detected using fluorescence activated cell sorter (FACS), immunohistology (immunohistochemistry of tissue sections), as well as substrate based detection assays such as ELISA, RIA or Western blotting or by using nucleic acid-based detection assays including in situ hybridization, RT-PCR and Northern blotting with probes specific for these markers.

The terms ***"level of expression"*** or ***"expression level"*** are used interchangeably and generally refer to the amount of a polynucleotide or a protein in a biological sample. According to the invention "expression" of a polypeptide, for example S100A8 and/or S100A9 proteins, may refer to transcription into a polynucleotide, translation into a protein, or even posttranslational modification of the protein.

It should be noted that the expression level is reflected by measurement and determination of an expression value. As used herein, the term "expression value", "level of expression" or "expression level" refers to numerical representation of a quantity of a gene product, which herein is a protein, but may also be an mRNA.

As used herein the term ***"comparing"*** denotes any examination of the amount or percent of MDSC or the expression level of any one of the suppressive characteristics of MDSC obtained in the samples of the invention as detailed throughout in order to discover similarities or differences between the measured values and a predetermined standard value or a value obtained in a control sample. It should be noted that comparing according to the present invention encompasses the possibility to use a computer based approach.

As used herein, the term ***"comprising"*** is intended to mean that the methods include the recited elements, but not excluding others.

The term ***"consisting of"*** when used to define methods, shall mean excluding other elements of any essential significance to the method.

In accordance with the present invention, there may be employed conventional molecular biology, microbiology, recombinant DNA, immunology, cell biology and other related techniques within the skill of the art. See, e.g., Sambrook et al., (2001) Molecular Cloning: A Laboratory Manual. 3rd ed. Cold Spring Harbor Laboratory Press: Cold Spring Harbor, N.Y.; Sambrook et al., (1989) Molecular Cloning: A Laboratory Manual. 2nd ed. Cold Spring Harbor Laboratory Press: Cold Spring Harbor, N.Y.; Ausubel et al., eds. (2005) Current Protocols in Molecular Biology. John Wiley and Sons, Inc.: Hoboken, N.J.; Bonifacino et al., eds. (2005) Current Protocols in Cell Biology. John Wiley and Sons, Inc.: Hoboken, N.J.; Coligan et al., eds. (2005) Current Protocols in Immunology, John Wiley and Sons, Inc.: Hoboken, N.J.; Coico et al., eds. (2005) Current Protocols in Microbiology, John Wiley and Sons, Inc.: Hoboken, N.J.; Coligan et al., eds. (2005) Current Protocols in Protein Science, John Wiley and Sons, Inc.: Hoboken, N.J.; Enna et al., eds. (2005) Current Protocols in Pharmacology John Wiley and Sons, Inc.: Hoboken, N.J.; Hames et al., eds. (1999) Protein Expression: A Practical Approach. Oxford University Press: Oxford; Freshney (2000) Culture of Animal Cells: A Manual of Basic Technique. 4th ed. Wiley-Liss; among others.

### EXAMPLES

The present invention is described further below in working examples which are intended to further describe the invention without limiting the scope therein.

### Example 1: Analysis of melanoma patients

### Material and Methods

**MDSC analyses:** The human MDCS phenotype as described herein, HLADR⁻CD33⁺CD11b⁺, was determined using flow cytometry of blood samples, immunohistology of tumor sections, and ELISA. Suppressive features of MDSC were analyzed by measuring NO, ROS, the S100A8/9 pro-inflammatory proteins, cleaved caspase 3 and MDSC suppressive activity on T cells (down regulating CD247 and SNX9 expression and impairing T cell proliferation). Suppressive features are characterized by elevated levels of NO and ROS, increased levels of the S100A8/9 pro-inflammatory proteins, low levels of cleaved caspase 3 and MDSC suppressive activity on T cells (down regulating CD247 and SNX9 expression and impairing T cell proliferation).

**Cryopreservation and thawing procedure:** Peripheral blood (whole blood) samples were tested as fresh upon lysing the erythrocytes, or were cryopreserved by mixing the sample with DMSO/FCS (freezing medium) and were transferred into cryovial tubes at different volumes (100, 200, 300 and 500µl/vial). The cryovial tubes were first stored in liquid N₂ containers. For analysis, whole blood samples were then thawed into 15ml tubes containing preheated medium (RPMI-1640). After one wash whole blood samples were resuspended in PBSX1 (50-100µl whole blood in 200µl PBSX1) and stored in 4° C refrigerators for maximum 1h prior to staining and flow-cytometry analysis or functional assays.

**Antibodies for surface and intracellular markers:** the following labeled anti-human monoclonal antibodies (mAbs) were used for staining: anti-HLA-DR-FITC, anti-CD27-FITC, anti-CD33-PE, anti-CD56-PE, anti-CD11b-APC, anti-CD3ε-APC, anti-HLA-DR-Pacific-blue (Biolegend). Rabbit polyclonal anti-human Cleaved caspase-3-Alexa Fluor 488 was purchased from Cell Signaling Technology. Prior to staining, each antibody was tittered to determine its optimal dilution using cryopreserved whole blood samples obtained from healthy donors.

**Staining whole blood samples:** for human myeloid derived suppressor cells (MDSCs) staining, whole blood samples were loaded on a 96U shape plates and washed twice with a flow stain buffer (PBSX1, 1% human serum). After adding anti-CD 11b, CD33 and HLA-DR mAb (50µl/well in flow stain buffer), whole blood samples were incubated for 30min at room temperature (RT) in the dark. After incubation with mAb 200µl of IX eBioscience-Step Fix/Lyse solution was added, mixed thoroughly and incubated for another 30min at RT in the dark, washed twice and resuspended in 200µl of flow stain buffer. For CD247 or cleaved caspase-3 intracellular detection, whole blood samples were loaded on 96U shape plates and washed twice with PBSX1. Samples were fixed with 2% paraformaldehyde in PBS for 20min at 4° C in the dark, washed twice with PBSX1 and permeabilized (with permeabilization buffer, PB-0.1% saponin, and 1% human serum) for 10min at RT in the dark. After washing the samples twice anti-CD56, CD3ε, CD247 or anti-CD33, CD11b, HLA-DR and Cleaved caspase-3 antibodies were added (50µl/well in PB) for 30min at 4°C in the dark. Samples were then washed twice with PB and were resuspended with 200µl flow stain buffer.

**Flow cytometry:** Samples were analyzed by FACSCalibur using Cell Quest software (BD). For MDSCs detection, after the initial FSC/SSC discrimination, the gate was set on HLA-DR⁻ cells and then on the CD11b⁺CD33⁺ population. To evaluate cleaved caspase-3 expression in MDSCs, the gate was first set on HLA-DR⁻ cells and then the percent and mean fluorescent intensity (MFI) of positive cells in the CD11b⁺CD33⁺ population was determined. For CD247 expression in T cells, the gate was first set on CD3ε⁺CD56⁻ cells and then the MFI of CD247 in this population was determined. Cleaved caspase-3 staining was performed using primary Rabbit anti-cleaved caspase-3 (Cell Signaling Technology) and secondary FITC-horse anti-Rabbit antibody (Thermo Scientific).

**ROS detection:** APF (Cell Technology) was used to measure ROS production by MDSC. Whole blood samples were incubated at 37° C in HBSS in the presence of APF and 5µg/well LPS for 30min. Un-stimulated and stimulated samples were then stained with labeled anti-CD33, CD11b and HLA-DR mAb in 50µl HBSS. After 30min incubation, 200µl of IX eBioscience-Step Fix/Lyse solution was added, mixed thoroughly and incubated for another 30min at RT. Samples were then washed twice with HBSS and analyzed by four color flow cytometry. Gating was the same as used for Cleaved caspase-3. Aliquots of Antibody-stained samples which were not incubated with APF served as controls.

**NO detection:** DAF-2DA (cell technology) was used to measure NO production by MDSC. Whole blood samples were incubated at 4° C in PBSX1 in the presence of DAF-2DA for 30min. Samples were then stained with labeled anti-CD33, CD11b and HLA-DR mAb in 50µl PBSX1. After 30min incubation, 200µl of IX eBioscience-Step Fix/Lyse solution was added, mixed thoroughly and incubated for another 30min at RT. Samples were then washed twice with PBSX1 and analyzed by four color flow cytometry. Gating was the same as used for Cleaved caspase-3. Aliquots of Antibody-stained samples, which were not incubated with DAF-2DA served as controls.

**Isolation of MDSCs:** Whole blood samples were treated with erythrocyte lysis buffer (ELB-eBioscience) to remove red blood prior to the isolation procedure. White blood cells were first incubated with anti-HLA-DR Ab-coated magnetic beads (Miltenyi) for 30min and separated using the MACS columns (Miltenyi) according to the manufacturer orders. Negatively selected HLA-DR⁻ cells were then incubated with anti-CD33 coated magnetic beads for 30min to positively identify CD33⁺ HLA-DR⁻ and was followed with another incubation for 30min with anti-CD 11b coated magnetic beads to positively identify CD33⁺CD11b⁺HLA-DR⁻ MDSCs.

**Statistical analysis:** Statistical analyses were performed using GraphPad Prism 5.04. Averaged values are presented as the mean ± s.e.m. When comparing two groups, statistical significance was determined using two-tailed Student's t test. When more than two groups were investigated, we performed an analysis of variance (ANOVA). Survival analyses were assessed using Geham-Breslow-Wilcoxon test. P values of less than < 0.05 were considered statistically significant.

### Results:

### HLA-DR ⁻CD33⁺CD11b⁺ MDSC levels are increased in melanoma patients.

The percentage of MDSC with the profile CD11b+CD33⁺HLA-DR⁻ was measured in peripheral blood samples (3-5ml) of healthy donors (n=40) and stage-4 melanoma patients (n=56) prior to ipilimumab (anti-CTLA4) treatment. All the melanoma patients were diagnosed with metastatic melanoma. The samples were taken according to the Helsinki approval. Clinical parameters were acquired from the medical records of patients.

The MDSCs were isolated and analyzed by FACS as described in materials and methods above. As can be seen in Figure 1A the percentage of MDSC in melanoma patients (the cancer patients) is significantly higher then that observed in healthy donors. Peripheral blood from the healthy donors and the melanoma-patients were also analyzed by flow cytometry for CD247 expression presented as mean fluorescence intensity (MFI), gating on CD3⁺ cells (Figure 1B). As seen in Figure 1C there is an inverse correlation between the percentage of MDSCs and CD247 expression presented as %MFI in the melanoma patients. A higher percentage of MDSC correlated with a lower expression of CD247. Down regulation of CD247 is an indication of suppressive activity on T cells.

### MDSCs levels are not affected by age, tumor origin or chemotherapeutic treatments prior to ipilimumab treatments.

In order to evaluate the effects of additional parameters on the level of MDSC in the blood of melanoma patients, various patient groups were analyzed. First, melanoma patients were divided into two age groups, 30-60 years old and 60-90 years old. Peripheral blood was obtained from the patients and the percentage of MDSCs in the blood was evaluated in these two age groups by using flow cytometry analysis as described in Materials and Methods above. As can be seen in Figure 1A the age group of the melanoma patient does not affect the level of MDSC in the peripheral blood. Similarly, as shown in Figure 2B the tumor origin (e.g. skin or ocular) also does not affect MDSC level. Interestingly, also the exposure to chemotherapeutic treatments (prior to ipilimumab treatments) did not seem to affect MDSC levels in these patients.

### MDSCs levels can distinguish between responders and non-responders to ipilimumab treatment.

In order to evaluate whether MDSC levels in the blood can predict responsiveness to treatment with the anti CTLA4 antibody ipilimumab, the levels of MDSC in the peripheral blood of melanoma patients were measured, as described above, prior to treatment with ipilimumab. Following treatment with ipilimumab, the melanoma patients were divided into two groups: a group of responders which included patients that manifested stable disease and patients that showed a complete response, and a group of non-responders which included patients with progressive disease. As shown in Figure 3A there was a strike correlation between high levels of MDSC in the peripheral blood of patients and subsequent failure to respond to treatment with ipilimumab. Clearly, this parameter could distinguish between the two groups of melanoma patients. In addition, the level of lactate dehydrogenase (LDH) was evaluated before the treatment with ipilimumab began. There was no significant difference between the groups of patients with respect to this parameter (Fig 3B).

### High MDSCs frequencies correlate with poor survival.

The following experiment was performed in order to evaluate whether MDSC levels in the blood can predict metastatic severity and patient survival. The levels of MDSC, as well as the levels of LDH, were measured in the peripheral blood of melanoma patients, as described above, prior to treatment with ipilimumab. As shown in Figure 4A, MDSCs levels are higher in patients with higher metastatic severity (M2), as compared with the group with lower metastatic severity (M1A/M1B/M1C). In addition, as shown in Figure 4B higher MDSCs levels are found in the group of patients with high LDH levels (LDH low<480 U/I; LDH high>480 U/I). In addition, the melanoma patients were divided into two groups according to their MDSCs levels measured prior to the ipilimumab treatments; one group was termed Low MDSCs (<55.5%, n=39) and the other group was termed High MDSCs (>55.5%, n=14). The percent survival and their survival in months were evaluated. As shown in Figure 4C patients with low MDSCs had a better survival curve than patients with high MDSCs. The survival in months of the Low MDSCs group being 11.56±1.2 (n=39) and of the High MDSCs group being 6.857±1.4 (n=14). Apparently, high MDSC levels are predictors of a more severe metastatic profile and of a shorter survival time of the cancer patient.

### LDH levels and metastatic severity correlates with patient's survival.

In this experiment the survival of melanoma patients was correlated with the severity of their metastatic stage and with their LDH levels. The patients were divided into two groups according to their metastatic level prior to ipilimumab treatments; the first group included patients with the relatively lower metastatic severity, namely M1A/B/C, and the second group included patients with the higher, M2, metastatic severity. As shown in Figures 5A and 5B the survival of patients in the M1A/B/C group was higher than the survival of the patients in the M2 group, being 12.4±1.2 months (n=35) in the M1A/B/C group and 6.1±1.1 months (n=18) in the M2 group. The patients were divided into two groups according their LDH levels prior to ipilimumab treatments; the first group included patients with the relatively lower LDH level, namely LDH<480 U/I; and the second group included patients with the higher LDH level, namely LDH>480 U/I. As shown in Figures 5C and 5D the survival of patients in the LDH low group was higher than the survival of the patients in the LDH high group, being 13.5±1.4 months (n=28) in the LDH low group and 6.6±0.9 months (n=20) in the LDH high group. Apparently, high LDH levels and a severe metastatic stage are predictors of a shorter survival time of the cancer patient.

### A combination of MDSCs. LDH and metastatic levels before ipilimumab treatments distinguishes between high and low survival rates of patients.

In view of the results shown in the two previous sections, in the following experiment the combined predictive value of measuring MDSC levels, LDH levels and metastatic severity on patients' survival was analyzed. Melanoma patients were divided into two groups according to their initial parameters as measured before the first ipilimumab treatment. Group I included patients with low levels of MDSCs, low levels of LDH and had metastatic staging of M1A-C (MDSC↓LDH↓M1A-C); group II included patients with high MDSCs levels, high LDH levels and a metastatic staging of M2 (MDSC↑LDH↑M2). The low and high levels were defined as shown in the above examples. As clearly shown in Figure 6 patients of Group I had a significantly higher survival rate as compared with the patients of Group II. Apparently, high MDSCs levels, high LDH levels and a severe metastatic stage are reliable predictors of a shorter survival time of the cancer patient.

### Analysis of ipilimumab responders

Table 1 shows parameters of 14 patients that responded to the ipilimumab treatment out of the 56 patients involved in this study. The Table shows the type of response (SD - stable disease; CR - complete response; PR - partial response; PD - progressive disease). Patient's number 12 and 14 had a very short period of responsiveness in comparison to the other responders, as indicated by their low survival rates (in months). These patients had high LDH levels on the day the first ipilimumab treatment was given and their staging level was M1C.

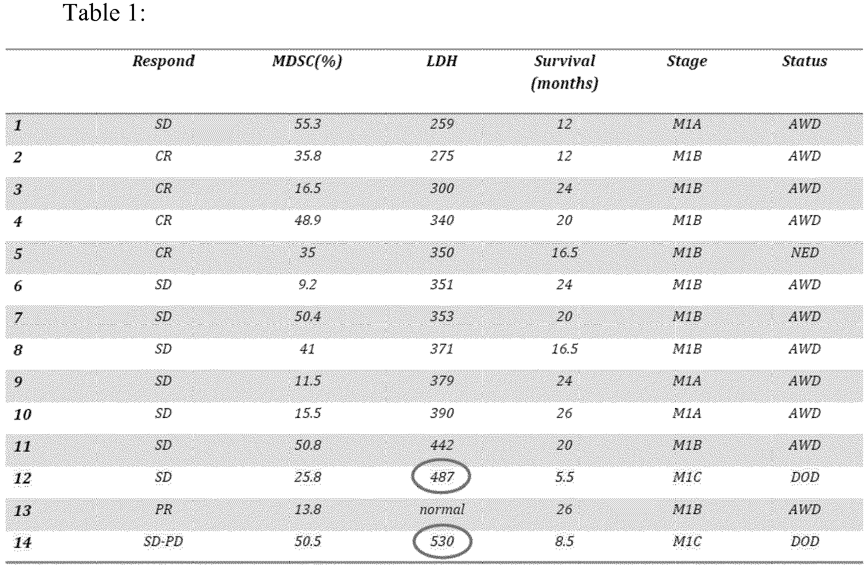

### Example 2: Analysis of metastatic colorectal cancer (CRC) patients and CRC mouse models

### Material and Methods

**Patients:** Peripheral blood samples were collected from 23 stage IV metastatic CRC-patients prior to and every 2 months in the course of chemotherapy treatments. All patients that were diagnosed with metastatic CRC underwent surgery and were not previously treated with chemotherapy. 20 healthy donors were used as controls. The samples were taken according to the Helsinki approval and analyzed for the indicated immune biomarkers in a "blinded test", not knowing the therapy specification. Following analyses completion, the specific treatment regiments and clinical parameters were acquired from medical records of the patients and correlation tests were performed.

**Mice**: Female C57BL/6 and BALB/c mice (aged 6-8 weeks) were purchased from Harlan (Jerusalem, Israel) and were grown at the Hebrew University specific-pathogen-free facility. All experiments were done in accordance with pre-approved institutional protocols.

***In vivo* mouse models: a model for colorectal cancer (CRC) and a model for tumor-free chronic inflammation:** Mice were injected intraperitoneally with 10mg/kg body weight of Azoxymethan (AOM, purchased from Sigma-Aldrich (St. Louis, MO, USA)) dissolved in physiological saline twice in two weeks intervals. Two weeks later, 2% dextran sulfate sodium (DSS, purchased from MP biochemicals Inc. (Santa Ana, CA, USA)) was given in the drinking water over 7 days, followed by 14 days of regular water (15). This cycle was repeated twice. Animals were sacrificed and analyzed three weeks after the last treatment. To induce a pathology-free chronic inflammation, a previously described protocol subjecting mice to heat-killed Mycobacterium tuberculosis (BCG) treatment (16) was used.

**CFSE staining and *ex-vivo* T-cell proliferation assay:** Splenocytes or purified T-cells isolated by a magnetic column separation system (Miltenyi Biotec) were labeled with 5µM CFSE (Invitrogen) and subjected to TCR-mediated activation as previously described (16).

***Ex** vivo* **myeloid-cell differentiation:** MDSCs were isolated from CRC and control (normal) mice and cultured in the presence or absence of 10ng/ml GM-CSF (PeproTech) for 3 days. In some samples, 5-fluoruracil (5FU) and Irinotecan (CPT11) were added to the cells, with or without GM-CSF, followed by phenotyping using flow cytometry.

**Flow cytometry analysis:** Isolated mouse splenocytes and peripheral blood lymphocytes (PBLs) were subjected to cell surface staining as previously described (16), using the following antibodies (Biolegend): FITC-labeled anti-Gr1, and anti-CD11c; PE-labeled anti-F4/80, anti-CD3ε, and anti-mNKp46; and biotinylated anti-CD 11b detected with streptavidin-Cy5. Intracellular staining for CD247 was performed as previously described (16) by using FITC-labeled anti-CD247 or biotinylated anti-CD247 (lone H146), the latter detected with streptavidin-Cy5. Foxp3 staining was performed according to the manufacturer's instructions (Miltenyi Biotec). Cleaved caspase-3 staining was performed using primary Rabbit anti-cleaved caspase-3 (Cell Signaling Technology, Asp175) and secondary FITC-horse anti-Rabbit antibody (Thermo Scientific). For intracellular NO⁻ and ROS detection, diaminofluoresciein-2 diacetate (DAF-2DA) reagent (NOS 200-1; Cell Technology) and aminophenyl fluorescein (APF) (4011; Cell Technology) were used respectively and determined by flow cytometry analysis.

For human whole blood cell phenotyping, intracellular staining of CD247 cells was performed by first fixing the cells with paraformaldehyde 1% followed by washes and permeabilized with 0.1% saponin. APC-labeled anti-CD11b and anti-CD3, PE-labeled anti-CD33, FITC-labeled anti-HLA-DR and anti-CD247 were used, all purchased from BD Pharmingen and used according to the manufacturer's protocol. After surface staining, cells were treated eBioscience-Step Fix/Lyse solution according to the manufacturer's instructions. All samples were analyzed using FACS Calibur with Cell Quest software (BD).

**Cell isolation from the colon:** The preparation of single cell suspensions from colons was performed using a modified version of a previously described protocol (17). Briefly, isolated colons were washed with HBSS 5%FBS (Invitrogen), digested, minced, incubated for 15 min at 37°C and epithelial cell suspension was washed with RPMI. For lamina propria cells, the retained tissue was transferred to collagenase/DNAse (Roche Diagnostic Corporation) solution, incubated for 1 h at 37°C, filtrated and washed with RPMI.

**Quantitative PCR analysis:** Total RNA was recovered from colon cells, splenocytes or isolated MDSCs and subjected to real-time PCR analysis as previously described (16). The sequences of the oligonucleotides used are listed in Table 2.

**Table 2:**

| Gene | Forward primer | Reverse primer |
|---|---|---|
| S100a8 | GGAAATCACCATGCCCTCTACAA SEQ ID NO:1 | ATGCCACACCCACTTTTATCACC SEQ ID NO:2 |
| S100a9 | GGAGCGCAGCATAACCACCATC SEQ ID NO:3 | GCCATCAGCATCATACACTCCTCA SEQ ID NO:4 |
| Hprt Hypoxanthine Phosphoribosy transferase | GTTAAGCAGTACAGCCCCAAA SEQ ID NO:5 | AGGGCATATCCAACAACAAACTT SEQ ID NO:6 |
| TNF-α | GCCACCACGCTCTTCTGTCTAC SEQ ID NO:7 | GGGTCTGGGCCATAGAACTGAT SEQ ID NO:8 |

**Western blot analysis:** Cells isolated from the spleen or colon were analyzed by Western blotting for the expression of various proteins as previously described (16). The antibodies used for immunoblotting were: anti-S100A9, anti-S100A8, and anti-aTubulin. Specific antibodies were detected by anti-rabbit or anti-goat antibodies conjugated to horseradish peroxidase (Jackson Immunoresearch), followed by enhanced chemiluminescence and exposure at blotting reader (Bio-Rad software).

**Histopathology and immunohistochemistry:** Paraffin-embedded colon tissue sections were prepared from CRC, CRC-5FU or CRC-CPT11 treated and control-untreated mice and stained with hemotoxylin and eosin solution. For immunohistochemistry, after antigen retrieval, sections were incubated at 4°C with primary antibodies: anti-β-catenin (BD) and anti-Gr-1 (Biolegend). For immunohistochemical staining, universal immuno-peroxidase polymer for mouse tissues (41431 IF; Histofine) was used, based on a horseradish peroxidase (HRP)-labeled polymer conjugated to anti-Rat. After incubation for 30 min, slide staining was completed by 3-5 min incubation with DAB+Chromogen (Lab Vision), followed by counterstaining with hematoxylin. As a control, samples were stained with each antibody and reagent individually.

**Statistical analysis:** Statistical analyses were performed using GraphPad Prism 5.04. Averaged values are presented as the mean ± s.e.m. When comparing two groups, statistical significance was determined using two-tailed Student's *t*-test. When more than two groups were investigated, an analysis of variance (ANOVA) was performed. Survival analyses were assessed using Fisher's exact test.

For the human experiments, paired *t*-test was used to compare samples from the same patients before and after FOLFOX or FOLFIRI treatment. Control and CRC groups were investigated by ANOVA.

### Results:

### FOLFOX and FOLFIRI therapies of CRC-patients display opposite effects on CD11b⁺CD33⁺HLA-DR⁻/ CD11lb⁺CD33⁺HLA-DR^{low} myeloid cells and immune status

The immune status of 23 stage IV metastatic CRC patients was assessed prior to receiving chemotherapy and compared with the immune status of 20 healthy donors. Peripheral blood samples were obtained from the healthy donors and the CRC patients and the levels of MDSC were measured using flow cytometry analysis of the cells. The percentage of CD11b⁺CD33⁺HLA-DR⁻ MDSCs in the patients' peripheral blood was significantly higher (12.65%±1.35%, *p*<0.01) as compared to healthy donors (5.35%±1.05%) (Fig.7A). Moreover a significant increased production of both NO⁻ (Fig. 7B) and ROS (Fig. 7C) by MDSCs was found in CRC patients as compared to healthy donors, showing their immunosuppressive features in the blood of CRC patients. In addition, an inverse correlation was found between the percentage of circulating MDSCs and CD247 expression in the CRC-patients (Fig. 7D), suggesting an impaired immune status associated with the disease. To examine the impact of FOLFOX and FOLFIRI on immune parameters of stage IV CRC patients, the effects of these drugs on the kinetics of MDSC levels and the association with CD247 expression were examined. All patients were treated with chemotherapy for at least six months. PBL analysis revealed decreased levels of circulating MDSCs following FOLFOX treatments (Fig. 7E), which were associated with a tendency of up-regulated CD247 expression (Fig. 7F). By contrast, during the course of FOLFIRI treatment of CRC patients the MDSC percentage continuously increased (Fig. 7G), correlating with CD247 down-regulation (Fig. 7H). These results suggest an adverse impact of the different chemotherapies on the CRC-patients' immune status.

### CPT11 but not 5-fluoruracil (5FU) treatment increases MDSC accumulation at the tumor site and support CRC growth

To further investigate whether 5FU and CPT11 display an adverse effect on the immune system, a mouse inducible CRC model based on AOM-DSS treatments was used (Fig. 8A). In this model, the development of CRC is accompanied by chronic inflammation and immune-suppression, reminiscent of spontaneous human CRC (18). Kinetic analysis of MDSC (defined as Gr-1+ CD11b⁺ cells) during CRC development revealed their gradual accumulation in the blood along with disease progression (Fig. 8B). When MDSC level became stable and adenomas were evident, mice were treated with either CPT11 or 5FU for three weeks. Histopathology of colonic late neoplasms developed in the AOM/DSS-treated mice was determined by hematoxylin-eosin stain. This analysis revealed that CPT11 monotherapy did not prevent tumorigenesis as no apparent differences in tumor loads within the colon were observed when compared to untreated CRC mice. This stood in sharp contrast to the dramatic effect of 5FU towards a decreased tumor load and recovery of colon architecture.
Moreover, 5FU and CPT11 were found to display opposite effects on β-catenin localization in CRC colons. Colons isolated from the normal and the untreated CRC-mice untreated or CRC-mice treated with 5FU or CPT11 were subjected to immunohistological staining with anti-β-catenin antibodies, as described in the methods. The immunohistochemical analysis showed a massive β-catenin accumulation in the nuclei of tumor cells both in colons from untreated and CPT11 treated CRC-mice, suggesting tumor progression (15).
Histological analyses of colons from CPT11 treated CRC-mice demonstrated not only a loss of entire crypts and surface epithelial layer, but also a massive leukocyte infiltration into the mucosa. Importantly, immunohistochemical probing of MDSCs within the colon revealed elevated levels of the cells in untreated and in CPT11 treated but not in 5FU treated CRC-mice. The same correlation between MDSC accumulation and the given treatment was also observed when testing tumors in colons. Analysis of cells generated from the colon lamina propria (LP) (Fig. 8C) and epithelium (Fig. 8D), depicted increased MDSC numbers in the CPT11 treated as compared to untreated CRC-mice. A significantly reduced MDSC infiltration was observed in both the LP (Fig. 8E) and epithelium (Fig. 8F) following 5FU treatment. Furthermore, a significant reduction of NO⁻ production was found in LP (Fig. 8G) and epithelial (Fig. 8H) MDSCs from 5FU treated CRC-mice, whereas after CPT11 treatment the NO⁻ levels remained elevated as compared to the CRC untreated mice. These results strengthen the CPT11 harmful effects supporting MDSC accumulation and suppressive features at the tumor site.

### CPT11 treatment increases systemic immunosuppression and counteracts 5FU beneficial effects

In this experiment, the effects of CPT11 and 5FU on the systemic immunosuppressive state were examined. As shown by the following experiment, a combined 5FU/CPT11 therapy abrogates recovery from immunosuppression during CRC progression. CRC-mice were either subjected to 5FU, CPT11 or a 5FU/CPT11 combination starting at week eight, or were left untreated. Three weeks after the second DSS treatment mice were sacrificed and spleens were analyzed. Untreated, CPT11- and 5FU/CPT11-treated CRC-mice display stronger inflammatory response as indicated by the enlarged spleen size as compared to 5FU treated CRC- or control-mice (Fig. 9A), and by the significantly decreased MDSC numbers in spleens of 5FU treated CRC-mice as compared to those of the untreated CRC-mice or those treated with CPT11 or 5FU/CPT11 (Figs. 9B and 9C). Interestingly, none of the monotherapies altered the high percentage of regulatory T-cells (CD4⁺Foxp3⁺Tregs) observed in CRC-mice (Fig. 9D), showing that mainly MDSCs are affected by 5FU and CPT11.

Moreover, while MDSCs from 5FU treated CRC-mice displayed a significantly reduced NO⁻ and ROS production, MDSCs from CPT11 or 5FU/CPT11 treated CRC-mice displayed elevated levels, as compared to untreated CRC-mice (Figs. 9E and 9F). MDSCs isolated from spleens of CRC-mice (n=5) were cultured *ex vivo* in the presence of 2.5µmol/L of 5FU, CPT11 or 5FU and CPT11 for 24h. The *Ex vivo* experiments showed that 5FU administration did not alter NO⁻ or ROS production in purified cultured MDSCs, but the addition of CPT11 or 5FU+CPT11 to the medium resulted in their elevation (Fig. 9G), suggesting a direct effect of the drugs on MDSC suppressive features. Both monocytic (CD11b⁺ Ly6C^{high} Ly6G⁻) and granulocyte (CD11b⁺ Ly6C^{low} Ly6G⁺) cell populations showed increased NO⁻ (Fig. 9H) and ROS (Fig. 9I) production upon CPT11 or 5FU+CPT11 treatment. However, the monocytic population displayed a more pronounced NO⁻ production, while granulocytic population showed more ROS production (Figs. 9G and 9H). Thus, CPT11 supports the immunosuppressive environment when applied alone or in a combination with 5FU, affecting the whole MDSC population.

Next, the effect of the given chemotherapies on the immune status was evaluated. This was performed by testing the function of the whole T-cell population (Fig. 9J) as well as the CD8⁺ T-cells (Fig. 9K) in all experimental groups along with measurements of the expression levels of CD247 in CD3⁺ (Fig. 9L) and in CD8⁺ T-cells (Fig. 9M). T-cell proliferative response was assessed by monitoring cell divisions of gated CFSE-labeled Thy1.2⁺ (CD90⁺) T-cells upon TCR-CD28 mediated activation. The results revealed a decreased T-cell proliferative ability following both CPT11 and 5FU/CPT11 treatments. In contrast, 5FU treatment of CRC-mice did not affect T-cell proliferation, as compared to untreated CRC-mice. Moreover, T-cell function was correlated with CD247 expression levels; low CD247 levels were obtained in T-cells from CRC-mice untreated and treated with CPT11 or a 5FU/CPT11 combination, while elevated levels were obtained upon 5FU treatment. Similar results were found in T-cells isolated from the LP and epithelium, inversely correlating with the local generated immunosuppressive environment (Figs. 9N and 9O). Thus, CPT11 and 5FU adversely affect not only the tumor but also the immune system, pointing at the dominating harmful effects of CPT11 when combined with 5FU.

### CPT11 harmful effects on the host's immune function are mediated via MDSCs

The observed MDSC elevation and increased tumor load in CPT11 treated CRC-mice as described above, suggests an impact of CPT11 on MDSC-induced cancer progression. Therefore, MDSC depletion could reduce the harmful effect of CPT11 on the immune status, and thereby enhance the anti-tumor effect. CRC-mice were subjected to either 5FU, CPT11 or a 5FU/CPT11 combination starting at week eight, or were untreated. At the same day of the second DSS administration, CPT11-treated CRC-mice were randomly separated into two groups; one group continued with CPT11 treatment while the second group was treated with anti-Grl mAb for MDSC depletion in addition to CPT11 treatment. A schematic description of the CRC mouse model in which the MDSCs were depleted by Gr1 mAb administration is shown in Fig. 10A. Three weeks after the second DSS treatment mice were sacrificed and PBLs (Fig. 10B) and colons (Fig. 10C) were analyzed. Indeed, *in vivo* MDSC depletion in the CPT11 treated CRC-mice, as indicated by the negligible MDSC levels as compared to CPT11 and 5FU/CPT11 treated CRC-mice (Fig. 10B), led to an almost complete regression of the tumors (Fig. 10C). Moreover, histopathological analysis using hematoxylin-eosin staining of colon sections revealed a differentiated adenocarcinoma in the colons of CRC-mice, CPT11 and 5FU/CPT11 treated CRC-mice, while only few aberrant crypt foci and tumors were detected in colons of 5FU treated CRC-mice and CPT11 treated MDSC depleted CRC-mice. This pattern indicates a beneficial effect of 5FU that attenuates CRC progression with a harmful contribution of CPT11, supporting immunosuppression and tumor progression via its effects on MDSCs. The daily recorded vitality and survival of the mice show a rapid deterioration, with increased death rates in CRC-mice treated with CPT11 or 5FU/CPT11, as compared to the 5FU, or CPT11 treated MDSC depleted CRC-mice, or even to untreated CRC-mice (Fig. 10D).

### MDSCs are insensitive to apoptosis under CPT11 treatment but become susceptible after 5FU treatment

Next, the mechanisms responsible for the opposite effects of 5FU and CPT11 on MDSC accumulation were investigated. One possible explanation for the observed differential effect of these drugs on MDSC levels could be a change in the sensitivity of the MDSCs to apoptosis, as previously reported for 5FU (11). Therefore, Splenic MDSCs from each group were analyzed for the expression of activated (cleaved) caspase-3 by flow cytometry analysis. To assess the direct effect of 5FU and CPT11 on cleaved caspase-3 expression, primary MDSCs isolated from spleens of CRC mice (n=6) were *ex-vivo* incubated with various doses of the drugs for 3 days and subjected to flow cytometry analysis. To assess which cells are affected by the chemotherapeutic drugs, MDSCs isolated from spleens of CRC mice were cultured *ex-vivo* with 10ng/ml of GM-CSF in the absence or presence of scaled-doses (0, 1.25, 2.5, and 5µmol/L) of 5FU or CPT11 for 3 days. Cleaved caspase-3 levels were then evaluated on the primary MDSCs, differentiated CD11c⁺CD11b⁺DCs and F4/80⁺CD11b⁺ macrophages. Indeed, a significant increased cleaved caspase-3 expression, an indicator for apoptosis, was observed within spleen MDSCs from 5FU treated CRC-mice, similar to that detected in MDSCs from control-mice (Fig. 11A). In contrast, following CPT11 or 5FU/CPT11 treatment, MDSCs displayed decreased cleaved caspase-3 levels, as in spleen MDSCs of untreated CRC-mice (Fig. 11A). Moreover, *ex vivo* studies revealed that 5FU but not CPT11 leads to cleaved caspase-3 up-regulation in purified cultured MDSCs in a dose-dependent manner (Fig. 11B and C). Interestingly, 5FU-induced apoptosis was evident only in non-differentiated MDSCs (Fig. 11D) while DCs (Fig. 11E) and macrophages (Fig. 11F) were insensitive, suggesting an exclusive effect of 5FU on the immature myeloid cell population.

In addition, 5FU and CPT11 were found to differently affect monocytic/granulocytic MDSC sensitivity to apoptosis. MDSCs isolated from spleens of CRC-mice (n=5) were cultured *ex vivo* in the presence of 2.5µmol/L of 5FU, CPT11 or 5FU and CPT11 for 3 days. Cultured MDSCs were analyzed for cleaved caspase-3 expression (MFI) by flow cytometry analysis gaiting on CD11b⁺Ly6C^{high} Ly6G⁻monocytic MDSCs (M-MDSCs) and CD11b⁺Ly6C^{low}Ly6G⁺ granulocytic MDSCs (G-MDSCs) sub-populations. It was found that 5FU controls both monocytic and granulocytic purified cultured MDSC populations, with the monocytic population being more sensitive, as indicated by the enhanced cleaved caspase-3 expression upon 5FU addition or when combined with CPT11 (Fig. 11G).

The drugs did not affect the apoptotic state of other immune cells such as T (CD3⁺) lymphocytes or B (B220⁺) lymphocytes (Figs. 11H and I). These results underscore the direct apoptotic effects of 5FU on immature MDSCs as opposed to the apoptosis insensitivity of MDSCs to the CPT11 or CPT11/5FU treatments.

### 5FU and CPT11 directly affect myeloid cell maturation and suppressive activity

Next, the effect of 5FU and CPT11 on MDSC maturation was examined. For that purpose the expression of the S100A8/9 pro-inflammatory proteins was assessed. The S100A8/9 pro-inflammatory proteins are induced in the course of tumorigenesis and chronic inflammation and play a role in controlling MDSC accumulation and retention in their immature suppressive state (7, 16, 19). S100A8/9 mRNA and protein levels were evaluated in MDSCs isolated from the spleen of CRC-mice, or CRC-mice treated with 5FU or CPT11 (n=4), α-Tubulin levels served as a control. While 5FU treatment of CRC-mice induced a significant decrease in S100A8/9 mRNA and protein levels in the spleen as compared to control-mice, increased S100A8/9 levels were observed following CPT11 treatment (Fig. 12A and B). Similar results were obtained when assessing the colon (Fig. 12C), suggesting that 5FU supports MDSC transition from an immature suppressive stage towards differentiated non-suppressive myeloid phenotype (16). Indeed, 5FU treatment of CRC-mice resulted in a significant shift towards differentiated DCs and macrophages (Figs. 12D and 12E) and to matured antigen presenting cells (APCs), shown by the induced CD80 and MHCII expression (Fig. 12F and 12G). In contrast, CPT11 treatment blocked myeloid cell differentiation *in vivo* (Figs. 12D-12G).

When testing the *ex vivo* direct effects of the drugs on GM-CSF mediated CRC-derived MDSC differentiation, it was found that, similar to the *in vivo* effects, CPT11 prevented cell differentiation after both 48h (data not shown) and 72h (Fig. 12H and 12I) as compared to MDSCs incubated with GM-CSF only. In contrast, 5FU enabled MDSC differentiation to DCs and macrophages (Fig. 12J and 12K). Interestingly, *ex vivo* CPT11-mediated MDSC differentiation blockade was associated with increased mRNA levels of the pro-inflammatory mediators TNFα (Fig 12L and 12M) S100A9 (Fig. 12N and 12O), while the 5FU-induced MDSC differentiation correlated with decreased levels of these factors. Thus, 5FU directly affects the differentiation pathway of MDSCs whereas CPT11 exhibits a differentiation blockade capacity when added to GM-CSF-treated MDSCs.

### 5FU and CPT11 opposing effects on MDSCs are tumor independent

Next, the immunoregulatory effects of 5FU and CPT11 were examined in a mouse model for chronic inflammation and associated immunosuppression (10), as described in the materials and method section above and in Fig. 13A. This experiment was performed in order to investigate whether the differential effects of the drugs are related to the presence of the tumor. Briefly, the mouse model for chronic inflammation was established by three repeated injections of heat-killed BCG bacteria. A day after the second BCG injection, mice were treated twice a week with 5FU, CPT11 or a 5FU/CPT11 combination. The 5FU beneficial and CPT11 harmful effects were similar to those observed in the CRC model. 5FU significantly reduced MDSC levels (Figs. 13B-13D) and ROS and NO⁻production (Fig. 13E and 13F). Treatment with 5FU also elevated cleaved caspase-3 levels (Fig. 13G), as compared to inflamed-untreated mice. In contrast, CPT11 or 5FU/CPT11 treatments induced opposite effects (Figs. 13B-G). No changes in CD4⁺Foxp3⁺ Tregs percentage were detected (Fig. 13H), confirming that these chemotherapies specifically affect MDSCs.

Next, it was assessed whether the 5FU and CPT11 opposite effects on MDSCs have different impacts on the host's immune competence. Assessment of the drugs' effects on total T-cell activity, and specifically CD8⁺ T-cells revealed a significant recovery of CD247 expression in the spleen and PBLs as well as T-cell proliferation following 5FU but not CPT11 or 5FU/CPT11 treatments (Figs. 13I-13L). Similar effects were also detected when analyzing NK-cells; down-regulated CD247 expression detected in NK-cells from chronically inflamed mice was almost completely recovered upon 5FU but not CPT11 treatment (Fig. 13M). Since NK-cell activity is mediated via natural cytotoxicity receptors (NCRs), which associate with and depend on CD247, NK-cell *in vivo* function was assessed by monitoring the clearance of adoptively transferred allogeneic cells. A complete recovery of NK-cell activity both in the spleen and PBLs was detected following 5FU treatment (Fig. 13N), along with a significant decreased clearance of allogeneic cells after CPT11 or 5FU/CPT11 treatments. These results indicate that 5FU and CPT11 opposite effects on MDSCs are tumor-independent, displaying a significant impact on effector T- and NK-cell responsiveness under chronic inflammatory conditions.

### References:

1. Terzic J, et al. Inflammation and colon cancer. Gastroenterology 2010; 138: 2101-14 e5.
2. Zhang B, et al. Circulating and tumor-infiltrating myeloid-derived suppressor cells in patients with colorectal carcinoma. PLoS One 2013; 8: e57114.
3. Sun HL, et al. Increased frequency and clinical significance of myeloid-derived suppressor cells in human colorectal carcinoma. World J Gastroenterol 2012; 18: 3303-9.
4. Coussens LM and Werb Z. Inflammation and cancer. Nature 2002; 420: 860-7.
5. Bruchard M, et al. Chemotherapy-triggered cathepsin B release in myeloid-derived suppressor cells activates the Nlrp3 inflammasome and promotes tumor growth. Nat Med 2013; 19: 57-64.
6. Bunt SK, et al. Inflammation enhances myeloid-derived suppressor cell cross-talk by signaling through Toll-like receptor 4. J Leukoc Biol 2009; 85: 996-1004.
7. Cheng P, et al. Inhibition of dendritic cell differentiation and accumulation of myeloid-derived suppressor cells in cancer is regulated by S100A9 protein. J Exp Med 2008; 205: 2235-49.
8. Kanterman J, et al. New insights into chronic inflammation-induced immunosuppression. Semin Cancer Biol 2012; 22: 307-18.
9. Ramakrishnan R, and Gabrilovich DI. Mechanism of synergistic effect of chemotherapy and immunotherapy of cancer. Cancer Immunol Immunother 2011; 60: 419-23.
10. Vaknin I, et al. A common pathway mediated through Toll-like receptors leads to T- and natural killer-cell immunosuppression. Blood 2008; 111: 1437-47.
11. Vincent J, et al. 5-Fluorouracil selectively kills tumor-associated myeloid-derived suppressor cells resulting in enhanced T-cell-dependent antitumor immunity. Cancer Res 2010; 70: 3052-61.
15. Popivanova BK, et al. Blocking TNF-alpha in mice reduces colorectal carcinogenesis associated with chronic colitis. J Clin Invest 2008; 118: 560-70.
16. Sade-Feldman M, et al. Tumor necrosis factor-alpha blocks differentiation and enhances suppressive activity of immature myeloid cells during chronic inflammation. Immunity 2013; 38: 541-54.
17. Drakes ML, et al. Isolation and purification of colon lamina propria dendritic cells from mice with colitis. Cytotechnology 2004; 46: 151-61.
18. De Robertis M, et al. The AOM/DSS murine model for the study of colon carcinogenesis: From pathways to diagnosis and therapy studies. J Carcinog 2011; 10: 9.
19. Sinha P, et al. Proinflammatory S100 proteins regulate the accumulation of myeloid-derived suppressor cells. J Immunol 2008; 181: 4666-75.

## Claims

1. A method for predicting and/or monitoring a cancer patient's response to treatment with an immunotherapeutic agent, said method consisting of the steps of:
**(a)** Measuring the amount of myeloid derived suppressor cells (MDSC) having the profile CD11b⁺ CD33⁺HLA-DR⁻ and/or CD11b⁺CD33⁺ HLA-DR^{low}, in at least one biological sample being a whole blood sample obtained from the patient, wherein said whole blood sample is unfractionated and a frozen sample or a cryopreserved sample; and
**(b)** Determining whether the amount of MDSC of said profile is higher than a predetermined standard value or higher than a control sample;
wherein
detection of a high amount of MDSC of the above profile in the at least one biological sample as compared with the predetermined standard value or the control sample indicates that the patient is not responding and/or will not respond or is poorly responding and/or will poorly respond to treatment with said agent.

2. A method according to claim 1 wherein said method is used for determining the patient's therapeutic regime by at least one of the following:
**(a)** Discontinuing treatment with said immunotherapeutic agent; or
**(b)** Combining the treatment with said immunotherapeutic agent with at least one additional compound being an anti-inflammatory and/or an anti-MDSC therapeutic agent.

3. The method of claim 1 wherein said measuring of step (a) is performed prior to treatment with said Immunotherapeutic agent.

4. The method of claim 1 or claim 2 wherein said measuring of step (a) is performed at least once, preferably more than once, during treatment with said immunotherapeutic agent.

5. A method according to any one of claims 1-4 wherein said immunotherapeutic agent is selected from the group consisting of therapeutic antibodies, immune checkpoints inhibitors cytokines, tumor infiltrating lymphocytes (TIL) and cancer vaccines.

6. A method according to claim 5 wherein said immunotherapeutic agents are anti CTLA4, anti PDL1 or anti PD1 agents.

7. A method according to claim 6 wherein said anti CTLA4 agent is the anti CTLA4 antibody Ipilimumab.

8. A method according to claim 6 wherein said anti PD1 agent is the anti PD1 antibody lambrolizumab.

9. A method according to any one of the preceding claims wherein the cancer is melanoma or colorectal carcinoma.

10. The method of any one of the preceding claims wherein said measuring of step (a) is a performed by a method comprising the step of contacting detecting molecules specific for MDSC with the biological sample.

11. The method of claim 10 wherein said detecting molecules are labeled detecting molecules.

12. The method of claim 10 wherein said detecting molecules are attached to a substrate.

13. The method of claim 10 wherein said detecting molecules are antibodies that specifically recognize and bind MDSC having the profile CD11b⁺CD33⁺HLA-DR⁻ and/or CD11b⁺CD33⁺HLA-DR^{low}.

14. The method of any one of the preceding claims wherein said method further comprises the step of at least one of:
**(a)** determining the expression levels of S100A8 and/or S100A9 proteins or encoding mRNA in said biological sample;
**(b)** determining the levels of cleaved caspase 3 in said biological sample;
**(c)** determining at least one of the level and/or activity of arginase 1 and iNOS in said biological sample;
**(d)** determining at least one of intracellular nitric oxide (NO) and reactive oxygen species (ROS) in said biological sample;
**(e)** determining the level of lactate dehydrogynase {LDH} in said biological sample;
**(f)** determining the level of MDSC suppressive activity on T cells in said biological sample.

15. A method for selecting a melanoma patient suitable for receiving treatment with an immunotherapeutic agent, said method consisting of the steps of:
**(a)** Measuring the amount of myeloid derived suppressor cells (MDSC) having the profile CD11b⁺CD33⁺HLA-DR⁻ and/or CD11b⁺CD33⁺HLA-DR^{low}, in at least one biological sample being a whole blood sample obtained from the patient, wherein said whole blood sample is unfractionated and a frozen sample or a cryopreserved sample; and
**(b)** Determining whether the amount of MDSC is lower than a predetermined standard value or lower than a control sample;
wherein
**(i)** said therapeutic agent is Ipilimumab or lambrolizumab or a combination thereof, and wherein
(ii) detection of a low amount of MDSC in the at least one biological sample as compared with the predetermined standard value or the control sample indicated that the patient is suitable for receiving treatment with Ipilimumab or lambrolizumab or a combination thereof.

## Patentansprüche

1. Verfahren zur Vorhersage und/oder Überwachung des Ansprechens eines Krebspatienten auf die Behandlung mit einem immuntherapeutischen Mittel, wobei das Verfahren aus den folgenden Schritten besteht:
(a) Messen der Menge von myeloid abgeleiteten Suppressorzellen (MDSC) mit dem Profil CD11b⁺ CD33⁺HLA-DR⁻ und/oder CD11b⁺CD33⁺ HLA-DR^{low} in wenigstens einer biologischen Probe, die eine von dem Patienten erhaltene Vollblutprobe ist, wobei die Vollblutprobe unfraktioniert und eine gefrorene Probe oder eine kryokonservierte Probe ist; und
(b) Bestimmen, ob die Menge von MDSC des Profils höher ist als ein vorbestimmter Standardwert oder höher als eine Kontrollprobe;
wobei
Nachweis einer hohen Menge von MDSC des obigen Profils in der wenigstens einen biologischen Probe im Vergleich zu dem vorbestimmten Standardwert oder der Kontrollprobe anzeigt, dass der Patient auf die Behandlung mit dem Mittel nicht anspricht und/oder nicht ansprechen wird oder schlecht anspricht und/oder schlecht ansprechen wird.

2. Verfahren nach Anspruch 1, wobei das Verfahren verwendet wird, um das therapeutische Regime des Patienten durch wenigstens eine der folgenden Maßnahmen zu bestimmen:
(a) Unterbrechen der Behandlung mit dem immuntherapeutischen Mittel; oder
(b) Kombinieren der Behandlung mit dem immuntherapeutischen Mittel mit wenigstens einer zusätzlichen Verbindung, die ein entzündungshemmendes und/oder ein anti-MDSC therapeutisches Mittel ist.

3. Verfahren nach Anspruch 1, wobei Messen von Schritt (a) vor der Behandlung mit dem immuntherapeutischen Mittel durchgeführt wird.

4. Verfahren nach Anspruch 1 oder Anspruch 2, wobei Messen von Schritt (a) wenigstens einmal, vorzugsweise mehr als einmal, während der Behandlung mit dem immuntherapeutischen Mittel durchgeführt wird.

5. Verfahren nach einem der Ansprüche 1 - 4, wobei das immuntherapeutische Mittel ausgewählt ist aus der Gruppe bestehend aus therapeutischen Antikörpern, Immun-Checkpoint-Inhibitoren, Zytokinen, tumorinfiltrierenden Lymphozyten (TIL) und Krebsimpfstoffen.

6. Verfahren nach Anspruch 5, wobei die immuntherapeutischen Mittel anti-CTLA4-, anti-PDL1- oder anti-PD1-Mittel sind.

7. Verfahren nach Anspruch 6, wobei das anti-CTLA4-Mittel der anti-CTLA4-Antikörper Ipilimumab ist.

8. Verfahren nach Anspruch 6, wobei das anti-PD1-Mittel der anti-PD1-Antikörper Lambrolizumab ist.

9. Verfahren nach einem der vorhergehenden Ansprüche, wobei der Krebs Melanom oder kolorektales Karzinom ist.

10. Verfahren nach einem der vorhergehenden Ansprüche, wobei Messen von Schritt (a) durch ein Verfahren durchgeführt wird, das den Schritt des Inkontaktbringens von für MDSC spezifische Nachweismoleküle mit der biologischen Probe umfasst.

11. Verfahren nach Anspruch 10, wobei die Nachweismoleküle markierte Nachweismoleküle sind.

12. Verfahren nach Anspruch 10, wobei die Nachweismoleküle an einen Träger angeheftet sind.

13. Verfahren nach Anspruch 10, wobei die Nachweismoleküle Antikörper sind, die spezifisch MDSC mit dem Profil CD11b⁺CD33⁺HLA-DR⁻ und/oder CD11b⁺CD33⁺HLA-DR^{low} erkennen und binden.

14. Verfahren nach einem der vorhergehenden Ansprüche, wobei das Verfahren weiterhin wenigstens einen der folgenden Schritte umfasst:
(a) Bestimmen der Expressionsspiegel von S100A8- und/oder S100A9-Proteinen oder Kodieren der mRNA in der biologischen Probe;
(b) Bestimmen der Spiegel von gespaltener Caspase 3 in der biologischen Probe;
(c) Bestimmen von wenigstens einem von den Spiegeln und/oder der Aktivität von Arginase 1 und iNOS in der biologischen Probe;
(d) Bestimmen von wenigstens einem von intrazellulärem Stickstoffmonoxid (NO) und reaktiven Sauerstoffspezies (ROS) in der biologischen Probe;
(e) Bestimmen des Spiegels von Laktatdehydrogynase (LDH) in der biologischen Probe;
(f) Bestimmen des Spiegels der MDSC-suppressiven Aktivität auf T-Zellen in der biologischen Probe.

15. Verfahren zur Auswahl eines Melanompatienten, der für die Behandlung mit einem immuntherapeutischen Mittel geeignet ist, wobei das Verfahren aus den folgenden Schritten besteht:
(a) Messen der Menge von myeloid abgeleiteten Suppressorzellen (MDSC) mit dem Profil CD11b⁺CD33⁺HLA-DR⁻ und/oder CD11b⁺CD33⁺HLA-DR^{low} in wenigstens einer biologischen Probe, die eine vom Patienten erhaltene Vollblutprobe ist, wobei die Vollblutprobe unfraktioniert und eine gefrorene Probe oder eine kryokonservierte Probe ist; und
(b) Bestimmen, ob die Menge von MDSC niedriger als ein vorbestimmter Standardwert oder niedriger als eine Kontrollprobe ist;
wobei
(i) das therapeutische Mittel Ipilimumab oder Lambrolizumab oder eine Kombination davon ist, und wobei
(ii) Nachweis einer geringen Menge von MDSC in der wenigstens einen biologischen Probe im Vergleich zu dem vorbestimmten Standardwert oder der Kontrollprobe anzeigt, dass der Patient für eine Behandlung mit Ipilimumab oder Lambrolizumab oder einer Kombination davon geeignet ist.

## Revendications

1. Procédé pour prédire et/ou surveiller la réponse d'un patient cancéreux à un traitement par un agent d'immunothérapie, ledit procédé consistant en les étapes consistant à :
(a) mesurer la quantité de cellules myéloïdes suppressives (MDSC) possédant le profil CD11b⁺CD33⁺HLA-DR⁻et/ou CD11b⁺CD33⁺HLA-DR^{low}, dans au moins un échantillon biologique qui est un échantillon de sang total obtenu chez le patient, dans lequel ledit échantillon de sang total est non fractionné et un échantillon congelé ou un échantillon cryoconservé ; et
(b) déterminer si la quantité de MDSC dudit profil est supérieure à une valeur standard prédéterminée ou supérieure à un échantillon de contrôle ;
dans lequel
la détection d'une quantité élevée de MDSC du profil ci-dessus dans le au moins un échantillon biologique par comparaison à la valeur standard prédéterminée ou à l'échantillon de contrôle indique que le patient ne répond pas et/ou ne répondra pas ou répond mal et/ou répondra mal au traitement par ledit agent.

2. Procédé selon la revendication 1, dans lequel ledit procédé est utilisé pour déterminer le régime thérapeutique du patient par au moins un des éléments suivants :
(a) l'interruption du traitement par ledit agent d'immunothérapie ; ou
(b) la combinaison du traitement par ledit agent d'immunothérapie avec au moins un composé supplémentaire qui est un agent thérapeutique anti-inflammatoire et/ou anti-MDSC.

3. Procédé selon la revendication 1, dans lequel ladite mesure de l'étape (a) est réalisée avant le traitement par ledit agent d'immunothérapie.

4. Procédé selon la revendication 1 ou la revendication 2, dans lequel ladite mesure de l'étape (a) est réalisée au moins une fois, de préférence plus d'une seule fois, pendant le traitement par ledit agent d'immunothérapie.

5. Procédé selon l'une quelconque des revendications 1 à 4, dans lequel ledit agent d'immunothérapie est sélectionné dans le groupe consistant en des anticorps thérapeutiques, des inhibiteurs de points de contrôle immunitaire, des cytokines, des lymphocytes infiltrant les tumeurs (TIL) et des vaccins contre le cancer.

6. Procédé selon la revendication 5, dans lequel lesdits agents d'immunothérapie sont des agents anti-CTLA4, anti-PDL1 ou anti-PD1.

7. Procédé selon la revendication 6, dans lequel ledit agent anti-CTLA4 est l'anticorps anti-CTLA4 ipilimumab.

8. Procédé selon la revendication 6, dans lequel ledit agent anti-PD1 est l'anticorps anti-PD1 lambrolizumab.

9. Procédé selon l'une quelconque des revendications précédentes, dans lequel le cancer est un mélanome ou un carcinome colorectal.

10. Procédé selon l'une quelconque des revendications précédentes, dans lequel ladite mesure de l'étape (a) est réalisée par un procédé comprenant l'étape consistant à mettre en contact de molécules de détection spécifiques pour des MDSC avec l'échantillon biologique.

11. Procédé selon la revendication 10, dans lequel lesdites molécules de détection sont des molécules de détection marquées.

12. Procédé selon la revendication 10, dans lequel lesdites molécules de détection sont attachées à un substrat.

13. Procédé selon la revendication 10, dans lequel lesdites molécules de détection sont des anticorps qui reconnaissent et se lient spécifiquement à des MDSC possédant le profil CD11b⁺CD33⁺HLA-DR⁻ et/ou CD11b⁺CD33⁺HLA-DR^{low}.

14. Procédé selon l'une quelconque des revendications précédentes, dans lequel ledit procédé comprend en outre l'étape consistant en au moins l'une de :
(a) la détermination des taux d'expression des protéines S100A8 et/ou S100A9 ou de l'ARNm codant dans ledit échantillon biologique ;
(b) la détermination des taux de la caspase 3 clivée dans ledit échantillon biologique ;
(c) la détermination d'au moins l'un du taux et/ou de l'activité de l'arginase 1 et d'iNOS dans ledit échantillon biologique ;
(d) la détermination d'au moins l'un de l'oxyde nitrique (NO) intracellulaire et des espèces réactives de l'oxygène (ROS) dans ledit échantillon biologique ;
(e) la détermination du taux de la lactate déshydrogénase {LDH} dans ledit échantillon biologique ;
(f) la détermination du niveau d'activité suppressive des MDSC sur les cellules T dans ledit échantillon biologique.

15. Procédé pour sélectionner un patient souffrant de mélanome approprié pour recevoir un traitement par un agent d'immunothérapie, ledit procédé consistant en les étapes consistant à :
(a) mesurer la quantité de cellules myéloïdes suppressives (MDSC) possédant le profil CD11b⁺CD33⁺HLA-DR⁻ et/ou CD11b⁺CD33⁺HLA-DR^{low}, dans au moins un échantillon biologique qui est un échantillon de sang total obtenu chez le patient, où ledit échantillon de sang total est non fractionné et un échantillon congelé ou un échantillon cryoconservé ; et
(b) déterminer si la quantité de MDSC est inférieure à une valeur standard prédéterminée ou inférieure à un échantillon de contrôle ;
dans lequel
(i) ledit agent thérapeutique est l'ipilimumab ou le lambrolizumab ou une combinaison de ceux-ci, et dans lequel
(ii) la détection d'une faible quantité de MDSC dans le au moins un échantillon biologique par comparaison à la valeur standard prédéterminée ou à l'échantillon de contrôle indique que le patient est approprié pour recevoir un traitement par l'ipilimumab ou le lambrolizumab ou une combinaison de ceux-ci.
